# EUROPEAN PATENT SPECIFICATION

(11) **EP 1 654 347 B1**
(45) Date of publication and mention of the grant of the patent: **04.06.2014**
(21) Application number: 04744911.1
(22) Date of filing: 27.06.2004
(51) Int. Cl.: C12M 3/00, C12N 5/00

(54) **Improved materials for constructing cell-chips, cell-chip covers, cell-chip coats, processed cell-chips and uses thereof**
Verbesserte Materialien zur Konstruktion von Zellchips, Zellchip-Abdeckungen, Zellchip-Hüllen, prozessierte Zellchips und Verwendungen davon
Matériaux ameliorés pour la construction de puces cellulaires, couvertures de puces cellulaires, revêtements de puces cellulaires, puces cellulaires traitées et leurs utilisations

(30) Priority: 26.06.2003 US 482437 P; 21.07.2003 US 488408 P; 05.11.2003 US 517073 P; 05.11.2003 US 517084 P; 17.02.2004 US 544356 P; 17.02.2004 US 544357 P
(43) Date of publication of application: 10.05.2006
(73) Proprietor: SENG ENTERPRISES LIMITED, Larnaca 7101 (CY)
(72) Inventor: DEUTSCH, Mordechai, 42 855 Doar Na Lev-HaSharon (IL); HERZBERG, Max, 76 834 Emeq Soreq (IL)
(74) Representative: Dennemeyer & Associates S.A.
(86) International application number: PCT/IL2004/000571
(87) International publication number: WO 2004/113492

(56) References cited:
- WO-A-02/48676
- WO-A-02/081662
- WO-A1-03/035824
- WO-A2-03/020871
- US-A- 4 308 351
- US-A- 4 729 949
- US-A- 4 839 280
- US-A- 5 627 045
- US-A- 6 103 479
- US-A1- 2002 173 033
- US-A1- 2003 030 184
- DANIEL J. TSCHUMPERLIN ET AL: 'Equibiaxial deformation-induced injury of alveolar epithelial cells in vitro' AM. J. PHYSIOL. vol. 275, 01 January 1998, pages L1173 - L1183, XP055029939
- LEE A A ET AL: "An equibiaxial strain system for cultured cells.", THE AMERICAN JOURNAL OF PHYSIOLOGY OCT 1996 LNKD- PUBMED:8897847, vol. 271, no. 4 Pt 1, October 1996 (1996-10), pages C1400-C1408, XP008152868, ISSN: 0002-9513
- GELEST: 'Optical Materials', [Online] 2007, Retrieved from the Internet: <URL:http://www.gelest.com/goods/pdf/SiCoat ings-2007.pdf> [retrieved on 2013-06-04]

## Description

The present invention relates to a chip-device for holding living cells according to claim 1, a method of making a chip device according to claim 5, and a method of growing cells according to claim 8.

The present invention relates to the field of cellular biology and more particularly, to an improved device for the study of cells as well as a method for producing the device. The present invention is also of methods for the study of cells, the methods implementable using devices of the present invention.

Combinatorial methods in chemistry, cellular biology and biochemistry are essential for the preparation of multitudes of active entities such as molecules. Once such active entities are prepared, it is necessary to study the effect of each of the active entities on living organisms. The study of the effects of active entities on living organisms is often performed on living cells. Cell-functions include many interrelated pathways, cycles and chemical reactions. Often, a study of an aggregate of cells, whether homogenous or heterogenous, does not provide interpretable results. Thus the comprehensive study of the effects of an active entity may require the examination of the effect of the active entity of single isolated living cells. Thus, the use of single-cell assays is one of the most important tools for understanding biological systems and the influence thereupon by various stimuli.

The combinatorial preparation of multitudes of active entities coupled with the necessity of studying the effects of all the active entitities using live-cell assays, requires the development of high-throughput methods for studying living cells, especially single live-cell assays.

In the art, various different methods for studying living cells are known.

Multiwell microtiter plates having 6, 12, 48, 96, 384 or even 1536 wells on a standard *ca*. 8.5 cm by *ca.* 12.5 cm footprint are well known in the art. The volume of the wells depends on the number of wells and the depthf thereof but generally is greater than 5x10⁻⁶ liter (for a 1536 well plate). Although exceptionally useful for the study of large groups of cells, multiwell microtiter plates are not suitable for the study of individual cells or even small groups of cells due to the large, relative to the cellular scale, size of the wells. Generally, cells held in such wells float about a solution in the wells and are not easily found for observation. When cells adhere to a well surface, the cells adhere to any location in the well, including anywhere on the bottom of the well and on the walls of the well. Such variability in location makes high throughput imaging (for example for morphological studies) challenging as acquiring an individual cell and focussing thereon is extremely difficult. Such variability in location also makes high-throughput signal processing (for example, detection of light emitted by a single cell through fluorescent processes) challenging as light must be gathered from the entire area of the well, increasing the noise in the signal. Further, cells held inside a large well of a microtiter plate can be physically manipulated only with difficulty. Thus, multiwell microtiter plates are in general only suitable for the study of large numbers of cells as a group.

In the art, a number of method and devices have been developed for the study of individual cells or a small number of cells as a group. Many such methods are based on using well-bearing devices. A well-bearing device is a device for the study of cells that has at least one well-bearing component for study of cells. A well-bearing component is a component having at least one, but generally a plurality of wells, each well configured to hold at least one cell. The term "well" is quite general and includes such features as dimples, depressions, tubes and enclosures. Since cells range in size from about 1 microns to about 100 (or even more) microns diameter there is no single well size that is appropriate for holding one cell of any type. That said, the dimensions of the typical individual well in the well-bearing components known in the art have dimensions of between about 1 microns up to about 200 microns, depending on the exact implementation. For example, a device designed for the study of single isolated 20 micron cells typically has wells of dimensions of about 20 microns. In other cases, larger wells are used to study the interactions of a few cells held together in one well. For example, a 200 micron well is recognized as being useful for the study of the interactions of two or three cells, see PCT patent application IL01/00992 published as WO 03/035824.

One feature that increases the utility of a well-bearing device is that each individual well is individually addressable. By individual adressability is meant that each well registered, found or studied without continuous observation. For example, after cells are held in wells of a well-bearing component, each cell is characterized and the respective well where each cell is held is noted. When desired, the observation component of the well-bearing device is directed to the location of the well where a specific cell is held. One method of implementing individual adressability is by the use of fiducial points or other features, generally on the well-bearing component. Another method of implementing individual adressability is by arranging the wells in a matrix of wells and finding a desired well by counting. Another method of implementing individual adressability is by providing a dedicated observation component for each well.

In the art, the well-bearing component of well-bearing devices is often a chip, a plate or other substantially planar component. Herein such a component is termed a "carrier". In the art, there also exist non-carrier well-bearing components of well-bearing devices, for example, bundles of fibers or bundles of tubes.

Mrksich and Whitesides, Ann. Rev. Biophys. Biomol, Struct. 1996, 25, 55-78; Craighead et al., J. Vac, Sci. Technol. 1982, 20, 316; Singhvi et al., Science 1994, 264, 696-698; Aplin and Hughes, Analyt. Biochem. 1981, 113, 144-148 and U.S. Patent 5,324,591 all teach of devices including matrices of spots of cell-attracting or cell-binding entities on a plate. In such devices, the spots serve as wells, binding to cells through a variety of chemical bonds. In such devices, the plate is the well-bearing component of the device. Due to the size of the spots, each such well generally holds more than one cell. To reduce interaction between cells held at different wells, the spots must be spaced relatively far apart, reducing loading as expressed in terms of wells per unit area. Even with wide spacing of wells, in such devices, cells are not entirely isolated from mutual interaction, nor can cells be subject to individual manipulation. The fact that the cells are not free-floating but are bound to the plate through some interaction necessarily compromises the results of experiments performed.

In U.S. Patent 6,103,479, the well-bearing component is a transparent carrier provided with a non-uniform pattern of wells, each well functionalized with chemical entities that bind to cells specifically or non-specifically. Each well is of approximately 200 to 1000 micron diameter and holds a plurality of cells. The interwell areas are hydrophobic so as not to attract cells. In addition to the carrier, a device of U.S. Patent 6,103,479 is provided with a chamber-bearing plate that mates with the carrier made of glass, plastic or silicon in which individually adressable microfluidic channels are etched. When brought together, the carrier and chamber-bearing plate constitute a casette in which each cell is bound to the carrier and isolated in a chamber provided with a fluid delivery system. Reagents are provided through the fluid delivery system and observed by the detection of fluoresence. In order to provide space for the walls of the chambers, the interwell areas of the carrier are relatively large, reducing loading as expressed in terms of wells per unit area. Subsequent to study, the cassette is separated into the two parts and the micro-patterned array of cells processed further. In some embodiments, the chamber-bearing plate is made of polytetrafluoroethylene, polydimethylsiloxane or an elastomer. As held cells do not make contact with the chamber-bearing plate it is not clear what advantages are to be had when providing a chamber-bearing plate of such esoteric materials.

In U.S. Patent 4,729,949 is taught a device for trapping individual cells in a well-bearing carrier, the carrier being substantially a plate having a plurality of individually adressable tapered apertures of a size to hold individual cells. Suction applied from the bottom surface of the plate where the apertures are narrow creates a force that draws cells suspended in a fluid above the carrier into the wide part of the apertures on the surface of the carrier to be held therein. Using the teachings of U.S. Patent 4,729,949 a specific group of cells (having dimensions similar to that of the aperture) can be selected from amongst a group of cells and held in the carrier. Although the cells are subjected to common stimuli, the fact that the wells are individually adressable allows the effect of a stimulus on an individual cell to be observed. A carrier of U.S. Patent 4,729,949 is generally made of metal and prepared using standard photoresist and electroplating techniques. In a carrier of U.S. Patent 4,729,949, the interwell areas of the carrier are relatively large, leading to a low loading as expressed in terms of wells per unit area.. Further, the suction required to hold cells in wells of a carrier of U.S. Patent 4,729,949 caused deformation of a held cell and makes a significant portion of the cell membranes unavailable for contact, both factors that potentially compromise experimental results. Study of cells with non-fluorescence based methods generally gives poor results due to reflections of light from the carrier.

In PCT patent application US99/04473 published as WO 99/45357 is taught a well-bearing device produced by etching the ends of a bundle of optical fibers (apparently of glass) to form a well-bearing component that is a bundle of fibers. The size of the hexagonal wells are demonstrated to be as small as 7 micron wide, 5 micron deep and have a volume of 1.45 x 10⁻¹³ liter. The interwell area is quite significant due to the thickness of the cladding of the optical fibers. Cells held in each well are independently observable through a respective fiber or by observation from above. In some embodiments, the inside surface of the wells is coated with a film of materials such as collagen, fibronectin, polylysine, polyethylene glycol, polystyrene, fluorophores, chromophores, dyes or a metal. Loading the well-bearing component of PCT patent application US99/04473 includes dipping the optical fiber bundle in a cell suspension so that cells adhere to the wells. There are a number of disadvantages to the teachings of PCT patent application US99/04473. The fact that the cells are studied only subsequent to adhesion to the wells necessarily influences the results of experiments performed. As cell proliferation starts soon after adhesion, it is never clear if a signal detected results from a single cell or a plurality of cells. It is is not clear where exactly in a well a cell is held and therefore what percentage of light emitted from a cell travels to a detector. The fact that emitted light travels through an optical fiber leads to loss of time dependent and phase information.

In PCT patent application IL04/000192 is taught a well-bearing device produced by bundling together glass capillaries, each glass capillary attached to an independent fluid flow generator such as a pump. A cell held in a first well is transferred to a second well by the simultaneous application of an outwards flow from the first well and an inwards flow into the second well.

In PCT patent application CH 02/00471, published as WO 03/020871, is taught a method for in vitro cultivation of cells, on a culture surface in the form of an expandable membrane. The cells are carried in a culture medium which is deposited on one side of the expandable membrane and the membrane is continually or periodically expanded by modifying the pressure on the opposite side of the membrane.

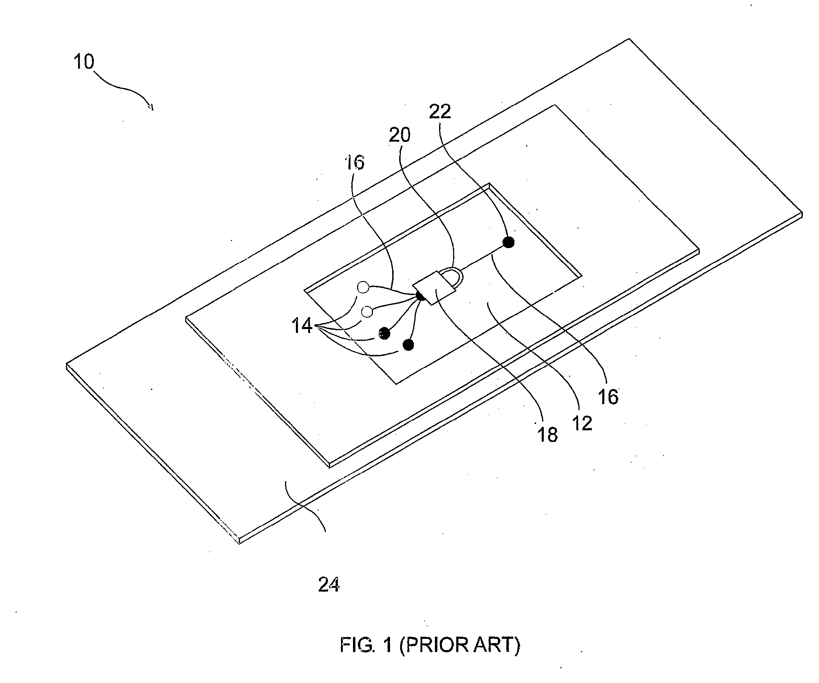

A preferred device for the study of cells is described in PCT patent application IL01/000992 published as WO 03/035824. The device **10**, depicted in Figure 1, is provided with a transparent carrier **12** as a well-bearing component. Carrier **12** is substantially a sheet of transparent material (preferably glass or polystyrene) on the surface of which features such as inlet connectors **14**, fluid channels **16**, wells (in Figure 1 a matrix of wells **18**), a fluid reservoir **20**, and an outlet connector **22**. Carrier **12** is immoveably held in a holder **24** having a cutout window of a size and shape to accept carrier **12**. Other components of device **10** not depicted include flow generators, observation components, external tubings and the like. When a cover slip (not depicted) is placed or integrally formed with over carrier **12**, fluid channels **16**, matrix of wells **18** and reservoir **20** are sealed forming channels that allow transport of fluids and reagents to cells held in matrix of wells **18**. The wells are configured to hold one or more cells and are preferably individually adressable both for examination and manipulation.

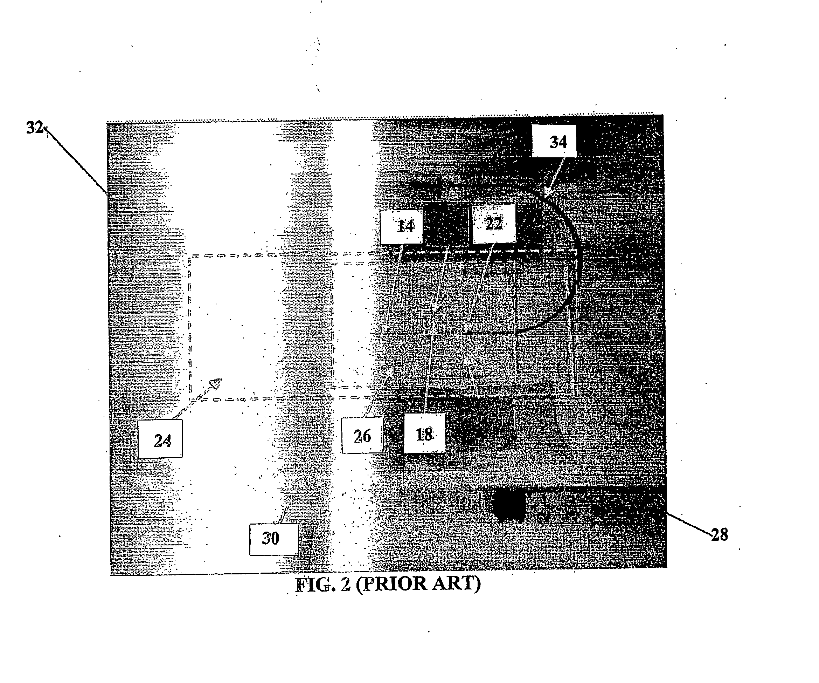

Figure 2 is a reproduction of a photograph of a different carrier **26** held in a holder **24**. A first syringe **28** as an inlet flow generator is in communication with an inlet connector **14** by a capillary tube **30**. Inlet connector **14** is in communication with matrix of wells **18** through a fluid.passage **16**. Matrix of wells **18** is in communication with outlet connector **22** through a fluid passage **16**. A second syringe **32** as an outlet flow generator is in communication with outlet connector **22** through capillary tube **34**.

PCT patent application IL01/000992 also teaches methods of physically manipulating cells held in a well-bearing device, using for example, individually addressable microelectrodes (found in the wells or in the cover slip) or optical tweezers. Typical physical manipulations include moving cells into or out of wells. One useful method that is implemented using a device of PCT patent application IL01/000992 is that cells, each held alone in a respective well, are examined (either in the presence or absence of reagents) and based on the results of the examination, cells with a certain characteristic are selected to remain in a respective well while cells without the certain characteristic are removed from a respective well and ejected by the application of a flow in parallel to the surface of the carrier, generated by a flow generator.

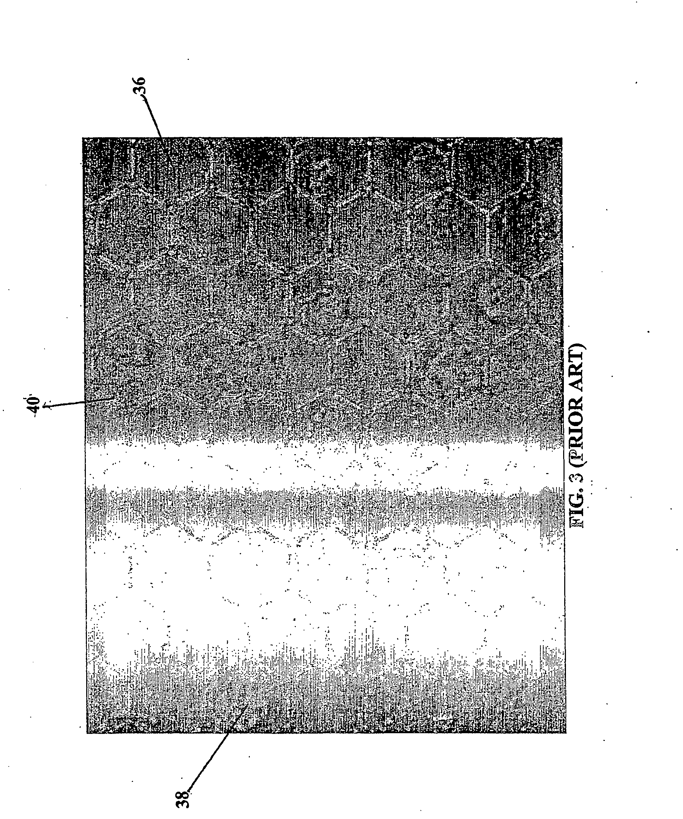

An additional feature of the teachings of PCT patent application IL01/000992 is that, in some embodiments, the area occupied by a well matrix is substantially entirely made up of wells with little or no interwell area, see Figure 3. Figure 3 is a reproduction of a photograph of part of a well matrix **18** from the top of a carrier **12** of PCT patent application IL01/00992. In Figure 3 is seen a plurality of hexagonal wells **36**, some populated with living cells **38**. It is seen that the interwell areas **40** make up only a minor percentage of the total area of well matrix **18**. This feature allows dense (near tissue density) cell packing, especially in single-cell well configurations and also allows simple well loading: a fluid containing suspended cells is introduced in the volume above the wells. Since there is little interwell area, cells invariable settle in wells.

One problem of the devices known in the art is that the materials from which the well-bearing components are made interfere with the study of cells. For example, in PCT patent application IL01/000992 the carrier is made of a transparent material such as glass or polystyrene. This is an excellent solution when studying cells by fluoresence. However, despite the transparency of the carrier, the fact that the index of refraction (n) of glass or polystyrene (n ∼ 1.5) is significantly greater that that of water or the physiological medium (n ∼ 1.33) in which living cells are found leads to scattering, reflection and diffraction of light, interfering with direct optical study of cells held in such carriers, for example, during morphological studies using a microscope. It would be advantageous to have a carrier of a material that is devoid of the problems associated with scattering, reflection and diffraction of light.

A further problem of the devices known in the art is that of proliferation of cells held or isolated in well-bearing components. Cells are held in wells. Movement of the well-bearing component causes cells to move out or be jostled from a well, leading to cell-loss or to cell-identity loss. Since proliferation takes time, this means that an entire device must be dedicated to studying one well-bearing component as long as there is interest in the cells held in the well-bearing component. This problem is solved in PCT patent application US99/04473 by encouraging cell-adhesion, but is suitable only for cells that are exceptionally adhesive and even then there is no guarantee that cells will not be lost. It has already been noted that in devices where cells are bound to wells, the actual binding may compromise experimental results. Further, there is often a desire to move or transport a cell-populated well-bearing component before cell adhesion has commenced. Further, even if the well-bearing component is not moved, proliferation of cells inside a well or an enclosure leads to unnatural population shapes, cell distortion and overcrowding effects. Further, if the cell populations grow outwards from an enclosure, the cells are subject to flow-induced loss or migration from the population itself to contaminate other wells. It would be advantageous to have a means that allows a cell-populated well-bearing component to be moved without concern that cells will exit respective wells. Such a means would preferably allow isolation, characterization, selection, proliferation and study of cells and at the same time allow storage, incubation and even transport of cells held therein without identity loss. Further, it would be advantageous to have a simple and efficient means to provide a plurality of cells that have been selected with enough space to proliferate without overcrowding effects.

A further problem of devices known in the art is that of fast cell proliferation. In some cases, cells held in wells proliferate quickly, before there is time to characterize the cells as individuals. This is a problem that often occurs when cells are first loaded onto a well-bearing component and stored with the intent of study at a later date, for example when a plurality of individual cells is held in a matrix wells for use as a biosensor or screening device. It would be advantageous to have a means that allows the study of cells using a well-bearing component such as is known in the field of cellular biology where cells are prevented, or at least delayed, from proliferating.

A further problem of devices known in the art is that of loading devices with cells from a sample. Generally, a sample of cells is suspended in a fluid and brought in proximity of the well-bearing component of a given device used to study the cells. One method of bringing the cell suspension in proximity of the well-bearing component is through the fluid channels. For example, in a device of Figure 1, a cell suspension is injected above well matrix **18** using first syringe **30**. Alternatively, the cover slip is removed, and a drop of cell suspension applied directly onto well matrix **18** and then the cover slip put back in place. By generating a force (*e.g*., by activating microelectrodes) to push or pull cells into wells or by allowing cells to settle by the force of gravity, cells populate the individual wells of well matrix **18**. Although such a loading method is suitable for cell suspensions such as blood or lymphatic fluid, when it is desired to study cells found in a solid matrix such as bodily tissue or an organ, the method if far from ideal. The step of releasing cells from a solid matrix is a time consuming and delicate operation. Often cells are killed by the process of release from the solid matrix. It would be advantageous to have means to harvest viable cells from a solid matrix for study using a well-bearing device.

It would be highly advantageous to have a device and methods for the study of cells not having at least some of the disadvantages of the prior art.

### SUMMARY OF THE INVENTION

The chip-device for holding living cells is defined in claim 1 of the present invention, the method of making a chip device is defined in claim 5 of the present invention, and the method of growing cells is defined in claim 8 of the present invention.

The present invention successfully addresses at least some of the shortcomings of the prior art by providing a new device, a method for producing the device, new methods for studying cells, and a new method for loading the well-bearing component of a well-bearing device.

According to the teachings of the present disclosure there is provided a chip-device for holding living cells, the device comprising a carrier having a plurality of wells disposed on a surface, each well configured to hold at least one (and preferably no more than one) living cell of a certain type, the device characterized in that the wells are configured to influence (preferably in a predetermined manner) the proliferation of living cells held in the wells. It is preferred that each of the wells is individually addressable. Each well has an "inside", that is a physical surface with which a cell held in the well may incidentally make contact.

In a preferred embodiment, the inside of the wells (with which held cells incidentally make contact) comprises a material selected from the group consisting of a gel, a hydrogel, polydimethylsiloxane, an elastomer, polymerized para-xylylene molecules, polymerized derivatives of para-xylylene molecules and silicon rubber.

In a preferred embodiment, the carrier is substantially made of a material selected from the group consisting of a gel, a hydrogel, polydimethylsiloxane, an elastomer and silicon rubber.

Typically configured devices of the present disclosure have at least one feature from amongst the six features:
(a) the inside of the wells is configured to delay cell proliferation;
(b) the inside of the wells is configured to inhibit cell proliferation;
(c) the wells are configured to allow cell proliferation inside and into at least one component of the chip-device;
(e) the inside of the wells is configured to delay adhesion of living cells thereto;
(f) the inside of the wells is configured to inhibit adhesion of living cells thereto; and
(g) the size of the wells is changeable.

In the present invention, the size of the wells of the carrier is changeable. The carrier is configured to be deformable in at least one dimension and upon deformation the size of at least one of the wells is increased. For example, upon deformation one, two or three dimensions of the carrier are changed, for example, the depth, the breadth, the length or a combination of any two or three of the dimensions.

In an embodiment of the present invention, the carrier is elastically deformable. Suitable materials from which to make an elastically deformable carrier include but are not limited to elastomers, rubbers and silicon rubbers.

In an embodiment of the present invention, the carrier is plastically deformable. Suitable materials from which to make an plastically deformable carrier include but are not limited to hydrocarbon wax, crystalline wax, polypropylene, isotactic polypropylene homopolymer, syndiotactic polypropylene homopolymer, metallocene catalyzed isotactic polypropylene homopolymer, metallocene catalyzed syndiotactic polypropylene homopolymer, ethylene-propylene random copolymer, butene-propylene random copolymer, ethylene-propylene-butene-1 terpolymer, low density polyethylene, isotactic polypropylene homopolymer, metallocene catalyzed syndiotactic polypropylene homopolymer, ethylene-propylene random copolymer, butene-propylene random copolymer, ethylene-propylene-butene-1 terpolymer, low density polyethylene, linear low density polyethylene, very low density polyethylene, metallocene catalyzed polyethylene, metallocene catalyzed polyethylene copolymers, ethylene-methacrylate copolymers, ethylene-vinyl acetate copolymers, ionomer resins, an ethylene-propylene random copolymer, ethylene-butene-1 copolymer, ethylene-propylene-butene-1 terpolymer, propylene-butene copolymer, low density polyethylene, linear low density polyethylene, very low density polyethylene, metallocene catalyzed polyethylene plastomer, metallocene catalyzed polyethylene, metallocene catalyzed polyethylene copolymers, ethylene-methacrylate copolymer, ethylene vinyl acetate copolymer, ionomer resin and combinations thereof.

In an embodiment of the present invention, the inside of the wells is configured to delay adhesion of living cells thereto. In an embodiment of the present invention, the inside of the well comprises a material that delays adhesion of living cells thereto, that is the carrier is substantially fashioned from the adhesion-delaying material or the inside of the wells is coated with the adhesion-delaying material. A suitable material to coat the inside of the well or from which to make a carrier comprises polydimethylsiloxane, is substantially polydimethylsiloxane or is substantially pure polydimethylsiloxane.

The dimensions of wells of a carrier of a chip-device of the present invention, depending on the specific embodiment, are less than about 200 microns, less than about 100 microns, less than about 50 microns, less than about 25 microns or even less than about 10 microns.

In the present invention, the wells are configured to hold no more than one living cell of a certain type.

According to the teachings of the present invention there is provided a method of making a chip-device, or other devices and carriers of the present invention comprising: (a) providing a template (such as a mold or stamp) having a negative of features of the surface of the carrier; (b) contacting the template with a precursor material so as to create the features in the precursor material; and (c) fixing the features in the precursor material so as to fashion the carrier as defined in claim 5.

Depending on the embodiment and the nature of the precursor material, fixing includes such methods a heating the precursor material, cooling the precursor material, polymerizing the precursor material, cross-linking the precursor material, curing the precursor material, irradiating the precursor material, illuminating the precursor material, gelling the precursor material, exposing the precursor material to a fixative and waiting a period of time.

The template is preferably made of a material that is rigid compared to the precursor material. Suitable materials include but are not limited to elastically deformable materials, plastically deformable materials, ceramics, epoxies, glasses, glass-ceramics, metals, plastics, polycarbonates, polydimethylsiloxane, polyethylenterephtalate glycol, polymers, polymethyl methacrylate, paraffins, polystyrene, polyurethanes, polyvinyl chloride, silicon, silicon oxide, silicon rubbers and wax.

Features created in the precursor material include such features as wells, channels, coupling elements, drains, fluid channels, fluid reservoirs, input ports, light sources, magnetizable elements, membranes, microreactors, microvalves, passages, optical components, optical fibers, optical filters, output ports, plumbing routes, protruberances, pumps, transport channels, valves, walls and fiducial points.

In an embodiment of the present invention the precursor material is a plastically deformable material (*vide infra*) such as a wax, a paraffin, plastic or polymer, and fixing the features simply includes separating the template from the precursor material.

In an embodiment of the present invention the precursor material is an elastically deformable material (*vide infra*) such as a gellable fluid, a polymerizable material, a powder, a fluid or a thermoplastic material.

In an embodiment of the present invention, the elastic precursor material is a thermoplastic material at plastic temperature and fixing the features includes cooling the thermoplastic material.

In an embodiment of the present invention, the elastic precursor material is a polymerizable material and fixing the features includes polymerizing the polymerizable material. Suitable polymerizable materials include but are not limited to monomer solutions, crosslinkable polymers, vulcanizable polymers, polymerizable fluid and thermosetting resins.

In a preferred embodiment, the polymerizable material is a polydimethylsiloxane precursor mixture and fixing the features includes polymerizing the polydimethylsiloxane precursor mixture so as to produce polydimethylsiloxane. In another preferred embodiment, the polymerizable material includes urethane and fixing the features includes polymerizing the urethane to produce polyurethane.

According to the teachings of the present disclosure there is also provided a method of making a chip-device, or other devices and carriers of the present invention comprising: (a) providing a carrier having a plurality of wells disposed on a surface, each well configured to hold at least one (and preferably no more than one) living cell of a certain type; and (b) coating the inside of the wells with a layer of a material configured to influence proliferation of living cells held in the wells.

In an embodiment of the present disclosure, coating the inside of the wells comprises (i) applying a precursor fluid to the inside of the wells; and (ii) solidifying the precursor fluid so as to form the proliferation-influencing layer. Suitable methods of solidifying include but are not limited to heating the precursor fluid, cooling the precursor fluid, polymerizing the precursor fluid, cross-linking the precursor fluid, curing the precursor fluid, irradiating the precursor fluid, illuminating the precursor fluid, gelling the precursor fluid, exposing the precursor fluid to a fixative and waiting a period of time.

In another embodiment of the present disclosure, coating the inside of the wells comprises (i) depositing a vapor of the material onto the surface thereby forming the proliferation-influencing layer.

In another embodiment of the present disclosure, coating the inside of the wells comprises (i) depositing a vapor of a precursor material onto the surface; and (ii) solidifying the precursor material thereby forming the proliferation-influencing layer. Suitable methods of solidifying include but are not limited to heating the precursor fluid, cooling the precursor fluid, polymerizing the precursor fluid, cross-linking the precursor fluid, curing the precursor fluid, irradiating the precursor fluid, illuminating the precursor fluid, gelling the precursor fluid, exposing the precursor fluid to a fixative and waiting a period of time. In a preferred embodiment, the vapor of precursor material is a vapor of para-xylylene molecules or derivatives thereof and the layer comprises the polymerized para-xylylene molecules (or derivatives thereof). By para-xylylene derivatives is meant a a molecule that is substantially a para-xylylene molecules having any additional substituent on either or both the aromatic rings

According to a feature of the present disclosure, the surface of the carrier is made of a material including but not limited to elastically deformable materials, plastically deformable materials, ceramics, epoxies, glasses, glass-ceramics, metals, plastics, polycarbonates, polydimethylsiloxane, polyethylenterephtalate glycol, polymers, polymethyl methacrylate, polystyrene, polyurethanes, polyvinyl chloride, silicon, silicon oxide and silicon rubbers.

The chip-devices of the present invention allow performance of a variety of heretofore difficult or impossible to perform experiments.

In an embodiment of the method of the present disclosure, the inside of the wells is a proliferation-delaying, such as a gel or a hydrogel. In an embodiment of the method of the present invention, the inside of the wells is an adhesion-delaying surface. Such a surface includes polydimethylsiloxane, is substantially polydimethylsiloxane or is substantially pure polydimethylsiloxane.

In an embodiment of the present disclosure, subsequent to gelling of the gel cover, at least one held cell is isolated by excising the at least one cell from the well-bearing component.

In one embodiment of the present disclosure, the gellable fluid includes an active entity. Suitable active entities include, but are not limited to antibodies, antigens, biological materials, chemical materials, chromatogenic compounds, drugs, enzymes, fluorescent probes, immunogenes, indicators, ligands, nucleic acids, nutrients, peptides, physiological media, proteins, receptors, selective toxins and toxins.

In one embodiment of the present disclosure, subsequent to gelling the gellable fluid, an active entity containing fluid is contacted with the produced gel cover. If the well-bearing component (such as a carrier) is also a gel, then an active entity containing fluid is also or exclusively contacted with the gel well-bearing component. Suitable active entities include, but are not limited to antibodies, antigens, biological materials, chemical materials, chromatogenic compounds, drugs, enzymes, fluorescent probes, immunogenes, indicators, ligands, nucleic acids, nutrients, peptides, physiological media, proteins, receptors, selective toxins and toxins. Subsequent to the contact of the active-entity, a period of time is waited so as to allow the active entity to diffuse through the gel cover (or gel well-bearing component).

In one embodiment of the present disclosure, subsequent to gelling the gellable fluid, the cells are allowed to proliferate into or through the produced gel cover. If the well-bearing component (such as a carrier) is also a gel, then the cells are allowed to proliferate into or through the gel well-bearing component. Although gels have cell-proliferation delaying properties, after some time cells do proliferate into and through gels.

According to the teachings of the present invention there is also provided a method of growing cells comprising: (a) providing a well-bearing device; (b) holding at least one and no more than one living cell of a certain type in a well of the well-bearing device of the present invention having a carrier with changeable well-sizes described hereinabove; and (c) increasing the size of the well so as to provide an increased space for proliferation of the cell as defined in claim 8.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRlEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1 (prior art) depicts a cell-chip device of PCT patent application IL01/000992 including a transparent carrier;
FIG. 2 (prior art) is a reproduction of a photograph of a cell-chip device of PCT patent application IL01/000992;
FIG. 3 (prior art) is a reproduction of a photograph of a cell-populated well matrix of a carrier of a cell-chip device of PCT patent application IL01/000992;
FIG. 4 is a reproduction of a scanning electron micrograph of the domes on a nickel stamp used for the production of a carrier of the present invention;
FIG. 5 is a reproduction of a scanning electron micrograph of a well-matrix of a polydimethylsiloxane carrier manufactured according to the method of the present invention using the nickel stamp of Figure 4;
FIGS. 6A-6C schematically depict a method of the present invention where a gel carrier is manufactured according to the method of the present invention and where a gel cover is manufactured according to the method of the present invention;
FIGS. 7A-7C schematically depict the use of an elastically deformable, carrier of the present invention;
FIGS. 8A-8D schematically depict the use of an elastically deformable carrier of the present invention;
FIG. 9A is a reproduction of a scanning electron micrograph of a well-matrix of a carrier of the present invention having sharp protuberances protruding from the surface of the carrier;
FIG. 9B is a reproduction of a scanning electron micrograph of a well-matrix of a carrier of the present invention having non-sharp protuberances protruding from the surface of the carrier; and
FIGS. 10A-10B schematically depict the method of collecting cells of the present invention.

### DETAILED DESCRIPTION OF THE INVENTION

The present invention is of a device that is substantially a well-bearing component for use in isolating cells, configured to influence the proliferation of living cells held in the wells as defined in the claims. The present invention is also of a method of producing a device of the present invention as defined in the claims. The present invention is also of a method of allowing cell proliferation by holding the cells in wells of a well-bearing component and then increasing the size of the wells as defined in the claims.

The principles and uses of the teachings of the present invention may be better understood with reference to the accompanying description, figures and examples. In the figures, like reference numerals refer to like parts throughout.

Before explaining at least one embodiment of the invention in detail, it is to be understood that the invention is not limited in its application to the details set forth herein. The invention can be implemented with other embodiments and can be practiced or carried out in various ways. It is also understood that the phraseology and terminology employed herein is for descriptive purpose and should not be regarded as limiting.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

The terms "comprising" and "including" or grammatical variants thereof when used herein are to be taken as specifying the stated features, integers, steps or components but do not preclude the addition of one or more additional features, integers, steps, components or groups thereof. This term encompasses the terms "consisting of" and "consisting essentially of".

The phrase "consisting essentially of" means that the composition may include additional ingredients, but only if the additional ingredients do not materially alter the basic and novel characteristics of the claimed compositions or methods.

The term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

Hereinfurther, the term "active entity" is understood to include chemical, biological or pharmaceutical entities including any natural or synthetic chemical or biological substance that influences a cell with which the entity is in contact. Typical active entities include but are not limited to active pharmaceutical ingredients, antibodies, antigens, biological materials, chemical materials, chromatogenic compounds, drugs, enzymes, fluorescent probes, immunogenes, indicators, ligands, nucleic acids, nutrients, peptides, physiological media, proteins, receptors, selective toxins and toxins.

Implementation of the methods of the present invention involves performing or completing selected tasks or steps manually, automatically, or a combination thereof.

**It is further to be undrstood that parts of the description below that do not correspond to claimed subject matter is presented for information only.**

### Device of the present invention

The invention provides a chip-device for holding living cells, the device comprising a carrier and a deforming device as defined in claim 1.

A device of the present disclosure is a chip-device for holding living cells resembling the chip device described in PCT patent application IL01/000992, the device including, together with other components, a carrier having a plurality of wells disposed on a surface of the carrier, each well configured to hold at least one (and preferably no more than one) living cell of a certain type, the device characterized in that the wells are configured to influence the proliferation of living cells held in the wells. Whereas prior art carriers may have had some characteristics that incidentally influence the proliferation of cells, in the present disclosure the influence is predetermined. Specifically desired features include that the inside of the wells is configured to delay (or inhibit) cell proliferation, to delay (or inhibit) cell adhesion, that the wells are configured so as to allow cell-proliferation inside and through at least one component of the chip-device, that the carrier has an index of refraction similar to that of water or that the size of the wells is changeable. A preferred means for implementing the teachings of the present disclosure is by coating the inside of the wells (with which held cells are potentially in contact) or by fashioning the carrier substantially in entirety from materials such as gels (especially hydrogels), polydimethylsiloxane or an elastomer such as silicon rubber.

As discussed hereinabove, a problem in the art is that there exists no simple way to allow cells that have been isolated and selected, for example according to the teachings of PCT patent application IL01/00992, to proliferate freely. On the one hand, for efficient selection and isolation, wells are necessarily small, too small to allow cells to proliferate therein. It is extremely difficult and inefficient to extract a single selected cell from a prior art well-bearing device and to place the cell in a sufficiently large location to allow proliferation. When, in accordance with the teachings of PCT patent application IL01/00992, a plurality of different cells are selected and isolated in one well-bearing device it is considerably more difficult to relocate each one of the plurality of cells to a remote location for proliferation.

Therefore, in the present invention, the size of the wells of a device of the present invention is changeable. The changeability of the wells is achieved by configuring a carrier of the present to be deformable in at least one dimension (length, breadth, depth, any two or all three) and that upon deformation, the size of at least one of the wells on the carrier is changed. Generally the deformation is by stretching and the change of size of a well is an increase of size of the well.

In one embodiment of the present invention the carrier is elastically deformable, for example, the carrier is made substantially of an elastically deformable material including but not limited to elastomers, rubber, silicon rubbers or other materials, for example elastic materials listed in U.S. Patent 6,740,727, U.S. Patent 6,682,792 and U.S. Patent 6,673,857. By elastically deformable material is meant a material that is capable of recovering shape after deformation. For example, a suitable elastomer that is commercially available is Silastic® LSR 9280-30 (Dow Corning Corporation, Midland, MI, USA). Such elastically deformable carriers are placed in a deforming device and by the application of tension stretched to a desired extent. The elastically deformable carrier remains in a deforming device and the tension maintained for as long as the changed size is desired.

In another embodiment of the present invention, the carrier is plastically deformable, that is the carrier is made of a plastically deformable material. By plastically deformable material is meant a material where the original topology is substantially maintained during deformation but does not recover shape after deformation. Such plastically deformable carriers are placed in a deforming device and by the application of tension stretched to a desired extent while topology such as surface features is substantially maintained throughout the deformation process. Tension can be released as the carrier does not recover the former shape. Plastically deformable materials useful in implementing a carrier of the present invention include but are not limited to hydrocarbon waxes (such as PARAFILM®, Pechiney Plastic Packaging, Inc., Neenah, WI, USA), crystalline wax, polypropylene, isotactic polypropylene homopolymer, syndiotactic polypropylene homopolymer, metallocene catalyzed isotactic polypropylene homopolymer, metallocene catalyzed syndiotactic polypropylene homopolymer, ethylene-propylene random copolymer, butene-propylene random copolymer, ethylene-propylene-butene-1 terpolymer, low density polyethylene, linear low density polyethylene, very low density polyethylene, metallocene catalyzed polyethylene, metallocene catalyzed polyethylene copolymers, ethylene-methacrylate copolymers, ethylene-vinyl acetate copolymers, ionomer resins, an ethylene-propylene random copolymer, ethylene-butene-1 copolymer, ethylene-propylene-butene-1 terpolymer, propylene-butene copolymer, low density polyethylene, linear low density polyethylene, very low density polyethylene, metallocene catalyzed polyethylene plastomer, metallocene catalyzed polyethylene, metallocene catalyzed polyethylene copolymers, ethylene-methacrylate copolymer, ethylene vinyl acetate copolymer, ionomer resin and combinations thereof.

The use of device of the present invention having changeable size wells is discussed in detail hereinbelow.

A further material that is used in producing a component of a device of the present disclosure for implementing the teachings of the present disclosure is a gel, especially a hydrogel. Suitable gels include but are not limited to hydrogels, agar, gelatin, agarose gels, low melting temperature agarose gels, alginate gels, roomtemperature Ca²⁺-induced alginate gels and polysaccharide gels. The components of the device that are advantageously made of gel are either a carrier, a cover for the carrier or both. Embodiments of the device of the present disclosure include embodiments where both the carrier and the cover aremade of gel. In such cases, the gel from which the carrier is made and the gel from which the cover are made may be substantially identical or may have different compositions. Suitable gels for the carrier, the cover or both include but are not limited to gels, hydrogels, agar gels, agarose gels, gelatins, low melting temperature agarose gels, alginate gels, roomtemperature Ca²⁺-induced alginate gels and polysaccharide gels. Embodiments of the device of the present disclosure include embodiments where both the cover is made of a gel (as described above) and the carrier is made of another material. Suitable materials from which carriers are made include but are not limited to elastically deformable materials, plastically deformable materials, ceramics, epoxies, glasses, glass-ceramics, metals, plastics, polycarbonates, polydimethylsiloxane, polyethylenterephtalate glycol, polymers, polymethyl methacrylate, paraffins, polystyrene, polyurethanes, polyvinyl chloride, silicon, silicon oxide, silicon rubbers and waxes.

The advantages of making components of a device from gels are manifold. Certain gels may have cell-proliferation delaying properties: a cell that is encased in such a gel does not significantly proliferate for a period of two to three days. However, once a cell begins to proliferate, proliferation occurs into and through the gel matrix with little interference. Thus a cell encased in a gel can proliferate without the overcrowding problems discussed hereinabove.

As discussed hereinabove, one problem of transparent devices known in the art is that the index of refraction of the materials from which the wells are made is significantly greater than that of water. For example glass has an index of refraction of 1.5 whereas water or physiological media have an index or refraction of only about 1.33. Considering the curvature and the dimensions of elements and features of well-bearing components of devices known in the art, the difference in index of refraction is significant enough to cause scattering, reflection and diffraction of light, interfering with optical study of held cells, for example, during morphological studies using a microscope. Thus, more generally, in an embodiment of the present disclosure the well-bearing component is substantially made of a material that has an index of refraction similar to the index of refraction of water or physiological medium. By an index of refraction similar to the index of refraction of water is meant herein an index of refraction of less than about 1.4, preferably less than about 1.38, more preferably less than about 1.36, more preferably less than about 1.35 and even more preferably less than about 1.34, or substantially identical to that of water.

Therefore, optical study of a cell through a component made of a transparent gel is exceptionally effective as gels, especially hydrogels, have an index of refraction substantially identical to the index of refraction of water and physiological media. As a result, when observing cells held in a gel, the gel components are substantially transparent and only the cells are seen. Gels that are substantially transparent to visible light, ultraviolet light and infrared radiation are known.

Although any type of gel can be used, hydrogels are preferred. Hydrogels are gels having a high percentage of water. Typical hydrogels useful for implementing the teachings of the present have a water content of greater than 80% by weight, greater than 92% by weight, greater than 95% by weight, greater than 97% by weight and even greater than 98% by weight.

As will be discussed in detail hereinfurther, in general a gel cover is advantageously produced by placing a gellable fluid over the top surface of a well-bearing component and subsequently gelled, sandwiching the cells between the wells and the gel cover. It is therefore preferred that a gel used be produced from a gellable fluid that is fluid and gels under conditions that are not damaging to a cell. Two exceptionally preferred types of hydrogels are alginates and low melting temperature agaroses.

Alginates are biologically compatible polysaccharide proteins that are fluid at low calcium ion concentrations (*e.g*., [Ca²⁺] < 1 µM) but gel upon exposure to higher concentrations of calcium ions (*e.g*., [Ca²⁺] = 20 mM). An exceptionally suitable alginate for implementing the teachings of the present invention is sodium alginate and may be purchased, for example, from Pronova Biopolymers (Drammen, Norway) as Protanal LF120 1% in water or Protanal LF200 1% in water.

Low melting temperature agaroses are biologically compatible gels that before gelling are fluid at temperatures that do not harm living cells (*e.g*., 20°C), gel at low temperatures that do not harm living cells (*e.g*., 4°C) and remain stable until well-above temperatures used for studying living cells (40°C). An exceptionally suitable agarose that may be purchased, for example, from Cambrex Bio Science Rockland Inc. (Rockland, ME, USA) is HGS-LMP Agarose (catalogue nr. 50221).

An additional advantage of a gel component, such as a carrier or a cover, is that it is possible to include an active entity, such as those discussed hereinabove, in the gel.

In a further embodiment, the inside of the wells (the physical surface of the well) of the carrier of a device of the present disclosure are configured to delay cell adhesion of living cells thereto. In one embodiment, the carrier is fashioned substantially from an adhesion-delaying material. In another embodiment, the carrier is fashioned from some material and the inside surface of the wells with which held cells potentially make contact is coated with an adhesion-delaying material.

In some embodiments of the present disclosure, a preferred material with which to coat a carrier or from which to make a carrier includes polydimethylsiloxane. When a coating material or the material from which the carrier is made includes polydimethylsiloxane, the polydimethylsiloxane is optionally one of the adhesion-delaying or inhibiting materials, is substantially the adhesion-delaying or inhibiting material or the material is substantially pure polydimethylsiloxane.

Substantially pure polydimethylsiloxane is a cross-linked polymer characterized by good optical transparency, low fluoresence, thermal and environmental stability and is inert to most laboratory reagents. Polydimethylsiloxane is not-cytotoxic. Importantly, polydimethylsiloxane has been found, to delay cell adherence, thus delaying cell proliferation, see below. Suitable polydimethylsiloxane resins are commercially available and can be purchased, amongst others, under the trade names RTV615 PDMS (GE Silicones, Wilton, CT, USA) and Sylgard 184 PDMS (Dow Corning Corporation, Midland, MI, USA).

A device of the present invention advantageously incorporates and includes many of the innovative features disclosed in PCT patent application IL01/000992. Preferred such features are discussed hereinfurther.

In embodiments of the device of the present invention it is preferred that each well be individually addressable.

In embodiments of the device of the present invention, the wells are defined by an intersection of at least two channels on the surface of the carrier. Preferably, the at least two intersecting channels are transport channels configured to transport fluids from one location of the carrier to another location of the carrier.

To increase loading of cells per unit area, it is preferred that the wells of a carrier of a device of the present invention be round or hexagonal and be hexagonally packed. Other preferred well shapes include square, triangular and rectangular wells.

In embodiments of the device of the present invention, the wells are juxtaposed. By juxtaposed is meant that in an area where wells are found, most of the area is well area and little of the area is interwell area. As disclosed in PCT patent application IL01/000992, when hexagonal wells are hexagonally packed, then a carrier can be fashioned so that the total interwell area between any cluster of seven wells is less than or equal to about 0.35, 0.25, 0.15, 0.10 or 0.06 the sum of the areas of the seven wells. This is more generally expressed herein in that the interwell area between two wells is less than or equal to 0.35, 0.25, 0.15, 0.10 or 0.06 of the sum of the areas of the two wells. In certain embodiments of the present invention it is preferred that the interwell area be substantially zero, that is that the rims of wells are substantially knife-edged.

In a typical example of a 2mm x 2mm matrix of hexagonal knife-edged wells of the present invention, where each well is about 10 microns wide, there are 61600 wells, a well density of about 1.5 x 10⁶ wells cm⁻¹.

The wells of a device of the present invention are generally of any size so as to hold one cell of a certain type. As the teachings of the present disclosure are directed to cellular biology, it is generally preferred that the wells be small so as to avoid having a large number of cells held in any one well. Thus, generally, the dimensions of the wells are generally less than about 200, 100, 50, 25 or even 10 microns. By dimensions is meant the usual meaning of the word and is dependent on the shape of the well. For example, for hexagonal or circular wells, the term dimension refers to diameter. For square or triangular wells is meant the longest dimension of the square or triangle, respectively. The exact size of wells of any given device is determined by the type of cells or alternately or additionally by the amount of cells to be studied using the device. Since different types of cells have different sizes, generally a device of the present invention will have wells of a. size to accommodate one cell of the type to be studies.

In embodiments of the device of the present invention, the bottoms of the wells of a carrier are preferably coplanar. This is exceptionally true when the wells are configured to hold only one cell of a certain type: coplanarity allows for optical observation of many cells (whether by scanning or simultaneously using a wide-angle observation component) without the need for time consuming and technically difficult to implement refocusing.

In some embodiments of the present invention, wells are dimples or depressions on the surface of the carrier. In other embodiments, the wells are substantially enclosures of dimensions such that substantially an entire cell of a certain type is containable within the enclosure, each enclosure having an opening at the surface, the opening defined by a first cross section of a size allowing passage of a cell of the certain type. The volume of such enclosure wells is typically less than 1 x 10⁻¹¹ liter (corresponding to the volume of a 200 micron cube), less than 1 x 10⁻¹² liter (corresponding to the volume of a 100 micron cube), less than 1 x 10⁻¹³ liter (corresponding to the volume of a 50 micron cube), less than 1 x 10⁻¹⁴ liter (corresponding to the volume of a 25 micron cube) and even less than 1 x 10⁻¹⁵ liter (corresponding to the volume of a 10 micron cube). In a preferred embodiment of the present invention, the dimensions of an enclosure are such as to contain no more than one cell of a certain size at any one time. The area of the first cross section, corresponding to the size of the opening of a respective enclosure is typically less than about 40000 micron² (corresponding to the area of a 200 micron square), 10000 micron² (corresponding to the area of a 100 micron square), 2500 micron² (corresponding to the area of a 50 micron square), 625 micron² (corresponding to the area of a 25 micron square) or even less than about 100 micron² (corresponding to the area of a 10 micron square).

In some embodiments, the surface of the carrier is substantially transparent so as to allow observation of cells while the lower surface is substantially not transparent. In some embodiments, the lower surface of the carrier is substantially transparent while the surface is substantially not transparent. In some embodiments, both the surface and the lower surface of the carrier are substantially transparent. In some embodiments, both the surface and the lower surface of the carrier are substantially not transparent. By transparent is especially meant transparent to one or more frequencies of electromagnetic radiation in the visible, ultraviolet or infrared spectra.

In some embodiments of the present invention, there is at least one fluid transport channel on the surface of the carrier, the fluid transport channel configured to transport fluids from one location of the carrier to another location of the carrier. In some embodiments of the present invention, there are fluid transport channels on the surface configured to transport fluids from one well to another well. In some embodiments of the present invention, a fluid transport channel separates one group of wells from another group of wells.

In some embodiments of the present invention there are channels for transporting fluids from the surface to the lower surface through the carrier. In some instances, the channels are pores in the wells (especially at the bottom of the wells), the pores being of a size so as to prevent passage of cell of the type to be studied therethrough.

In some embodiments of the present invention a device of the present invention has a cover slip as a component. For use, a cover slip is positioned above and generally substantially parallel with the surface of a respective carrier. The cover slip provides a closed volume and seals fluid transport channels and such-like features of the carrier so that fluids can be directed to flow as desired. In some embodiments a cover slip is attached to a respective carrier. Attaching is performed, for example, using an adhesive or a surface treatment such as plasma treatment. There exist many suitable adhesives, including but not limited to light curable adhesives, for example light curing adhesive 3051 or 3441 manufactured by Henkel Loctite Deutschland GmbH, München, Germany.

In a preferred embodiment, the device, the cover slip and the carrier are configured so as to allow the cover slip to removeably rest above the surface of the carrier substantially in parallel to the surface of the carrier. In some embodiments, the cover slip and the carrier are configured so that there exist only a limited number (*e.g*., six, four, three, two or even one) of "correct" cover slip positions where the cover slip is substantially oriented in a specific position when resting above the surface. Such a limited number depends on the shape and design of features of the carrier and specifically the shape and arrangement of the wells thereof.

There exist many reasons to design a cover slip having only a limited number of orientations. In a preferred embodiment, a cover slip is provided with one or more cover slip microelectrode, as detailed in PCT patent application IL01/000992. A cover slip microelectrode is generally configured to be positioned substantially above and associated with a specific well and, when activated, apply a repulsive force so as to push cells downwards into the associated well or apply an attractive force so as to extract cells out of the associated well. Clearly, for a cover slip microelectrode to be properly positioned and properly addressable, the cover slip preferably has only a limited number of correct positions.

In some embodiments of the present invention, a carrier is provided with a wall or walls, similar to walls detailed in PCT patent application IL01/000992. The details of shape and geometry of such a wall is dependent on the purpose such a wall serves. The wall may be configured to encircle all of the wells or to isolate groups of wells. Such a wall can be continuous or not. The top of the wall can define a plane. In some embodiments, such a wall acts as a mold to assist in making a gel cover. The wall surrounds the wells. When a gel cover is made by pouring a gellable fluid onto the surface of the carrier (*vide infra*), the wall holds the gellable fluid in place until the gellable fluid gels. In other embodiments of the present invention, such a wall helps support a cover slip in the proper position, orientation and height above the carrier. In other embodiments of the present invention, such a wall defines, together with a cover slip, a volume containing one or more wells.

In some embodiments of the present invention a carrier is provided with protuberances protruding from the surface, generally between two adjacent wells. The details of shape and geometry of such protuberances is dependent on the purpose such protuberances serve.

In some embodiments of the present invention, protuberances help support a cover slip in the proper position, orientation and height above the carrier.

In some embodiments of the present invention it is desired that when the cover slip is in place, fluids flow freely in the volume between the carrier and the cover slip, but cells are prevented from doing so. In such an embodiment it is often advantageous to provide protuberances between wells so that the size of the passage defined by the cover slip, the protuberances and the carrier between wells is such that a cell cannot pass therethrough acting, in fact, as a porous barrier to cell movement or fence.

In other embodiments, protuberances are used to implement the method of collecting cells of the present disclosure (*vide infra*). In brief, a biological sample is placed directly on a carrier provided with protuberances following the removal of a cover slip, if present. The protuberances assist the release of living cells from the biological sample. Cells released from the sample settle directly into wells of the carrier. Protuberances used for implementing the method of cell collection of the present disclosure are of any height that is convenient for production. Typical protuberances used for implementing the method of cell collection of the present invention are between about 1 micron high and about 20 microns high. Such protuberances can be sharp or not sharp. The term not sharp is a relative term, and depends on the dimensions of the cells to be harvested from the biological sample. It has been found that sufficiently not sharp so as not to pierce a cell under the conditions used but still effectively assist in removal of the cell from the biological matrix, a not sharp protuberance is generally of a width between about 5% and about 30% of the cell diameter, or preferably between about 10% and about 20% of the cell diameter.

A device of the present invention is advantageously provided with a flow generator configured to generate a flow of fluid substantially parallel to the surface of the carrier. As discussed in PCT patent application IL01/000992, a parallel fluid flow is useful for washing away cells that are not held in wells.

A device of the present invention may be used in conjunction with or is advantageously provided with optical tweezers and similar devices, configured to move cells found in the proximity of a carrier of the present invention. Optical tweezers can be used to push cells into wells or to extract cells therefrom.

The innovative use of gel as a cover as disclosed herein is not limited to a device of the present invention or to use with a carrier of the present invention. Rather, the use of a gel is useful for any device for holding living cells where the device includes a well-bearing component having a plurality of wells disposed on a surface. Suitable devices include but are not limited to all the devices discussed in the introduction hereinabove such as the well-bearing components taught in U.S. Patent 4,729,949, PCT patent application US99/04473, PCT patent application IL04/000192 and PCT patent application IL01/000992. It is important to note that the property of gels to allow cell proliferation therein or the property of gels to delay cell-proliferation are in some embodiments of secondary importance to the gel cover. The teachings of the present disclosure concerning a gel cover are applicable and useful not only due to the influence of the gel on the proliferation of living cells, but also for the use of the gel as a cover, preventing cells held in a well-bearing component from moving or being lost.

The gel cover can be implemented to cover a well-bearing component having wells of generally any size. As the teachings of the present disclosure are directed to cellular biology, it is generally preferred that the wells be small so as to avoid a large number of cells from being held in any one well. Thus, generally, the dimensions of the wells are generally less than about 200, 100, 50, 25 or even less than about 10 microns. The exact size of wells of any given well-bearing component is determined by the type of cells to be studied using the well-bearing component. Since different types of cells have different sizes, generally a well-bearing component covered with a gel cover will have wells of a size to accommodate one or more cells of the type to be studied Most preferred is that a well be of a size so as to hold no more than one cell of the type to be studied at any one time. Also preferred is that a well be of a size so as to hold a predetermined number of cells of the type to be studied.

Types of suitable gels preferred for implementing a gel cover are as discussed hereinabove. In a preferred embodiment, the gel used as a gel cover is substantially transparent (whether to visible light, ultraviolet light, infrared radition or some combination thereof). In some embodiments of the present disclosure it is desirable to include an active entity in a gel cover.

### Methods of manufacture of a device of the present invention

The invention provides a method of making a chip-device as defined in claim 5.

With the exception of the carrier, a chip-device of the present disclosure is produced using methods with which one skilled in the art is acquainted and described, for example, in PCT patent application IL01/000992.

A carrier is produced using any of a variety of methods known in the art. Suitable methods include methods that employ one or more techniques including but not limited to casting, embossing, etching, free-form manufacture, injection-molding, microetching, micromachining, microplating, molding, spin coating, lithography or photo-lithography.

The preferred method of producing a carrier is the method of the present disclosure. The method of the present disclosure for producing a carrier is substantially by providing a template having a negative of the features of the surface of the carrier. The template is brought in contact with a precursor material, thus creating the features of the carrier in the precursor material. The features are subsequently fixed in the precursor material, thus producing the carrier. Depending on the precursor material, fixing includes, but is not limited to, methods such as heating the precursor material, cooling the precursor material, curing the precursor material, polymerizing the precursor material, cross-linking the precursor material, irradiating the precursor material, illuminating the precursor material, gelling the precursor material, exposing the precursor material to a fixative and waiting a period of time. By fixative is meant an agent that causes the precursor material to change to the fixed state and is used herein as a general term for such materials as fixatives, hardeners, polymerization/ crosslinking / curing initiators, catalysts and agents. It is important to note that in some cases a precursor material is produced by mixing two or more components which thereafter change to a fixed state, for example, by simply waiting a period of time.

The template having a negative of the features is, for example, a stamp or a mold, and is made of any suitable material that is more rigid than a respective precursor material, including but not limited to elastically deformable materials, plastically deformable materials, ceramics, epoxies, glasses, glass-ceramics, metals, plastics, polycarbonates, polydimethylsiloxane, polyethylenterephtalate glycol, polymers, polymethyl methacrylate, paraffins, polystyrene, polyurethanes, polyvinyl chloride, silicon, silicon oxide, silicon rubbers and wax.

The template is made, for example, using methods with which one skilled in the art is acquainted such as casting, embossing, etching, free-form manufacture, injection-molding, microetching, micromachining, microplating, molding, lithography or photolithography. The features created in the precursor material by the contact of the template include the wells and additional features such as drains, channels, coupling elements, drains, fluid channels, fluid reservoirs (having U-shaped or V-shaped profiles), input ports, light sources, magnetizable elements, membranes, microreactors, microvalves, passages, optical components, optical fibers, optical filters, output ports, plumbing routes, pumps, transport channels, valves, and fiducial points. Features also include protruberances for separating wells from each other, protruberances for supporting a cover, protruberances for implementing the methods of the present disclosure, walls and partial walls.

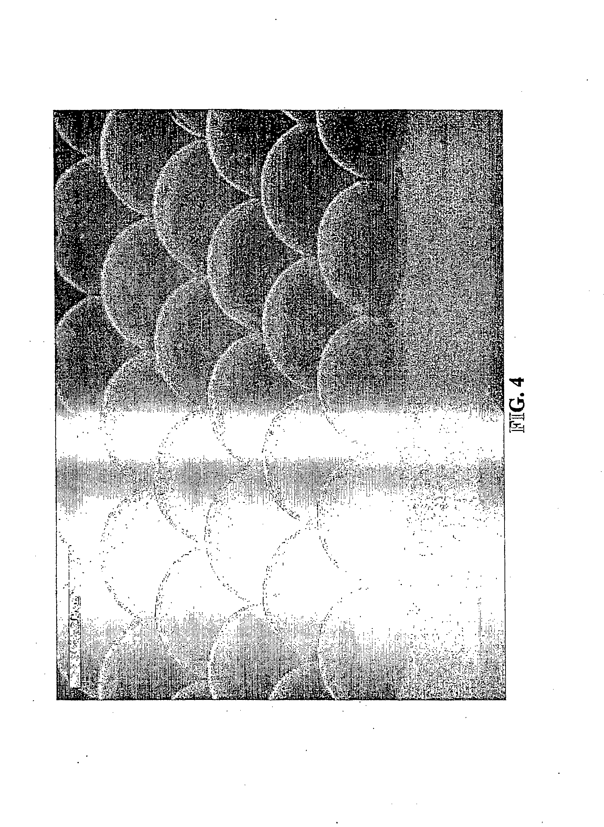

In Figure 4, is shown a reproduction of a scanning electron micrograph of the domes on a nickel stamp used as a template for the production of a carrier. Seen is an array of hexagonally-packed domes that are the negative of a hexagonal array of knife-edged wells. The diameter of the domes at the intersection with the nickel surface is approximately 20 microns.

An important feature created is a feature that is used as a fiducial point, or a number of features each used as a fiducial point. In one preferred embodiment of the present disclosure, a fiducial point is a feature having a special or distinct shape. In a preferred embodiment, when a fiducial point is made according to the method of the present disclosure, a marking material (*e.g*., a fluorescent material such as fluorescein), a visible material or a metal) is added to the fiducial point, especially before the features are fixed in the precursor material. The preferred method of adding a marking material is by applying the material to the respective negative of the fiducial point on the template. When the template is removed at least some of the marking material stays in the thus-formed fiducial point.

Once the features are fixed and the carrier produced, the template is separated from the carrier, the carrier cut to size if necessary and the chip-device assembled by attaching the carrier to other chip-device components. Addition chip-device components include a cover slip, piping, tubing, pumps, fluid supplies, observation components and the like. In some embodiments, the additional chip-device components, especially the cover slip, are attached to the carrier using, for example, adhesives or surface treatments such as anodic bonding, fusion bonding or plasma treatment such as plasma discharge (exceptionally suitable for polydimethylsiloxane, see Duffy et al., Anal. Chem. 1998, 70, 4974-4984).

In one preferred embodiment of the present invention, the precursor material is a plastically deformable precursor material. Examples of plastically deformable precursor materials include waxes, paraffins, plastics, polymers and the like. In a preferred embodiment, the template is a stamp, and the contacting of the template with the precursor material is substantially stamping the features of the carrier onto the precursor material, preferably under controlled thermal conditions. In such cases, the precursor material and the material from which the carrier are generally chemically substantially similar and there is no need for a separate action to fix the features in the precursor material beyond separating the produced carrier from the template.

In another preferred embodiment of the present invention, the precursor material is an elastic precursor material. Herein by elastic precursor material is meant a material that is capable of recovering shape after deformation and includes gellable fluids, polymerizable materials, powders, fluids and thermoplastic materials.

In a preferred embodiment, the elastic precursor material is a thermoplastic material at an elastic temperature (*e.g*., when moldable or molten). Subsequent to the contacting of the template but before the contact is finished, the thermoplastic material is cooled, thus fixing the desired features in the incipient carrier.

In another preferred embodiment, the elastic precursor material is a polymerizable material (*e.g*., monomer solutions, crosslinkable polymers, vulcanizable polymers, polymerizable fluids, or thermosetting resins). Subsequent to the contacting of the template but before the contact is finished, the polymerizable material is polymerized, thus fixing the desired features in the incipient carrier. In such cases, the precursor material and the material from which the carrier is made are chemically dissimilar (for example, have the relationship of monomer to polymer).

One preferred polymerizable precursor material is a non-cured polydimethylsiloxane precursor mixture. A mixture of two polydimethylsiloxane components (the prepolymer and curing agent) are mixed together in the desired ratio (preferably about 10:1, but ratios between about 5:1 and about 20:1 are generally suitable) to give a polydimethylsiloxane precursor mixture, the mixture degassed and contacted with the template. The features are fixed by the curing of the mixture. Curing of polydimethylsiloxane precursor generally takes place at room temperature for about 24 hours and, when desired, is accelerated by hating. For example it has been found that carriers of the present invention made of polydimethylsiloxane are ready for further processing within 2 hours when cured at 60°C or within 15 minutes when cured at 150°C. A detailed review of methods for the production of micronic features on polydimethylsiloxane suitable for implementing the teachings of the present invention are known in the art and discussed, for example, in Ng et al., Electroplaoresis 2002, 23, 3461-3473 and Duffy et al., Anal. Chem. 1998, 70, 4974-4984.

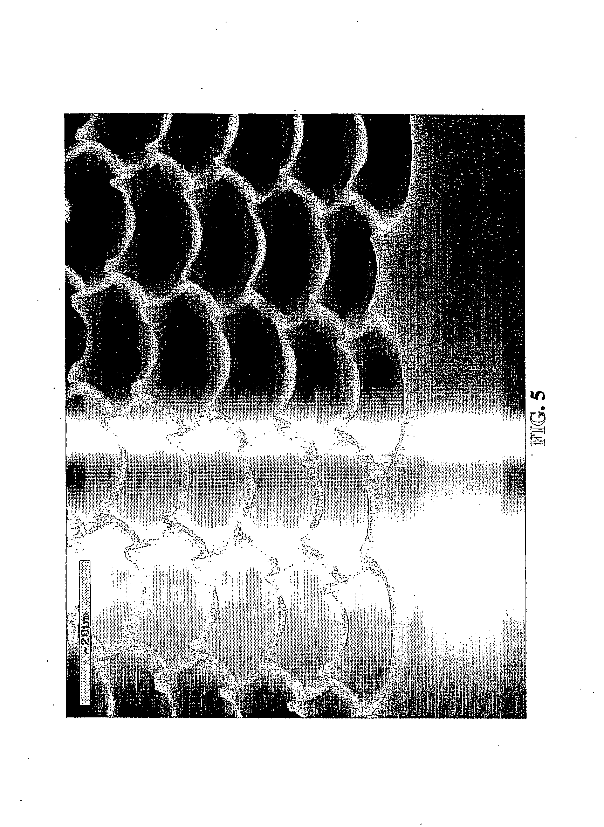

In Figure 5 is shown a reproduction of a scanning electron micrograph of a well-matrix of a polydimethylsiloxane carrier manufactured as described herein using the nickel stamp depicted in Figure 4.

Another preferred polymerizable precursor material is urethane that is polymerizable to yield polyurethane.

Another preferred elastic precursor material is a gellable fluid. After the gellable fluid is brought in contact with the template, the features are fixed by gelling the gellable fluid to yield a gel. Most preferred are gellable fluids that produce a hydrogel.

Methods for gelling gellable fluids known in the art include fluids that gel upon heating, fluids that gel upon cooling, fluids that gel upon irradiation or illumination, fluids that gel as a result of contact with a gelling reagent or fluids that gel after a period of time. Preferred gellable fluids include solutions of proteins, alginates, protein polysaccharides and low melting temperature agaroses.

One preferred gellable fluid is a low-melting temperature agarose solution. Such a solution is fluid at temperatures that do not harm living cells (*e.g*., 20°C) and gel at low temperatures that do not harm living cells (*e.g*., 4°C). An exceptionally suitable agarose for implementing the teachings of the present invention that may be purchased, for example, from Cambrex Bio Science Rockland Inc. (Rockland, ME, USA) is HGS-LMP Agarose 0.5% in PBS (catalogue nr. 50221).

Another preferred gellable fluid is an alginate solution which gels upon contact with a gelling reagent, the preferred gelling reagent being a solution having a Ca²⁺ ion concentration of greater than about 1 x 10⁻⁶ M. An exceptionally useful gelling agent is a 20 x 10⁻³ M calcium gluconate solution. Suitable alginate solutions can be purchased from Pronova Biopolymers (Drammen, Norway) and include, for example, Protanal LF 120 1% in water and Protanal LF200 1% in water.

A minor technical difficulty occasionally noted during the application of a gel cover is that during the gelling step of the gel cover, the gel contracts leaving a small gap in the carrier / gel cover interface. It has been found that such gaps are adequately filled by the addition of additional gellable fluid followed by gelling of the fluid.

Another preferred method of making a carrier is by photolithography of a spin-coated substrate, a commercially available process (for example, from Micro Resist Technology GmbH, Berlin, Germany) known to one skilled in the art. According to such a method, a high aspect ratio photoresist fluid (*e.g*., SU-8 thich photoresist, MicroChem Corporation, Newton MA, USA) is placed on a planar substrate (for example a glass slide). The substrate is rotated horizontally, that is, about an axis that is perpendicular to the surface of the substrate on which the photoresist fluid was placed. As a result of the rotation the photoresist fluid forms a uniformly thick layer on the substrate, typically between about 5 microns and about 20 microns thick. Subsequently, the photoresist fluid is illuminated through a mask, fixing only desired areas of the substrate layer. Developing of the substrate with the selectively fixed film layer removes the non-fixed areas of the film. In such a way a carrier of the present invention is produced made up of a fixed photoresist layer resting on a substrate layer where the features of the carrier are carved into the photoresist layer and the bottom of the features (such as wells) is the substrate. Using photolithography, flat-bottomed wells and other features are easily produced. Such a method is a preferred method of producing a two-layered carrier of the present disclosure.

Some embodiments include a carrier made of some material where the wells thereof are coated with a layer that influences the proliferation of living cells, for example delaying or preventing cell proliferation, for example by delaying or preventing adhesion of cells held in the wells.

The material of which a carrier having coated wells according to the teachings of the present disclosure is made is any material used in making carriers and includes but is not limited to elastically deformable materials, plastically deformable materials, ceramics, epoxies, glasses, glass-ceramics, metals, plastics, polycarbonates, polydimethylsiloxane, polyethylenterephtalate glycol, polymers, polymethyl methacrylate, polystyrene, polyurethanes, polyvinyl chloride, silicon, silicon oxide and silicon rubbers.

One skilled in the art is acquainted with many ways to coat the insides of wells of a carrier so as to provide a carrier of the present invention having a coating that influences the proliferation of cells held therein.

One preferred method of coating the insides of wells of a carrier, applicable to virtually any carrier produced by virtually any method, is by vapor deposition. Vapor deposition involves the deposition of materials such as molecules or atoms onto a surface at low pressures and is characterized by the production of evenly thin coatings on a surface, such as the surface of a carrier. A preferred coating for implementing the teachings of the present invention is made of polymerized para-xylylene molecules (or derivatives thereof) deposited by vapor deposition, a coating commercially known as Parylene®. Parylene® is preferred not only for cell adhesion delaying properties but also for the fact that Parylene® coatings are uniform, thin (typically 0.1 - 1 micron) and without voids even when the coated surface includes configurations with sharp edges, points, flat surfaces, crevices or exposed internal surfaces.

### Experimental methods implented using the device of the present invention

The invention provides a method of growing cells as defined in claim 8.

The teachings of the present disclosure provide the possibility of applying heretofore difficult or impossible methods for manipulating cells in the field of cellular biology. Some of the methods are discussed hereinbelow.

A first method of manipulating cells involves holding cells in wells of a well-bearing component and then covering the held cells with a gel cover. As is discussed in the hereinabove, there exist many cell-holding well-bearing components. Such components include the well-bearing components of devices taught in U.S. Patent 4,729,949, PCT patent application US99/04473, PCT patent application IL04/000192 and PCT patent application IL01/000992. Preferred, however, is to hold cells in the wells of a carrier. For ease of observation, it is preferred that the well-bearing component be transparent.

Generally, a plurality of cells is held in the wells of a well-bearing component and a gellable fluid is placed in proximity of the wells, so as to fill the wells without displacing the cells held therein. It is often covenient to first mix the cells in the gellable fluid, place the cell/gellable fluid mixture in proximity of the wells and subsequently to cause the cells to settle into the wells so as to be held in the wells. Settling of cells can occur simply due to gravity, or can be performed, for example, by centrifugation of the cells together with the wells. As is discussed hereinabove and in PCT patent application IL01/000992, it is preferred that the wells be juxtaposed. When the wells are justaposed, the cells settle only in wells and not on the interwell areas.

The gellable fluid is subsequently gelled so as to form a cover. As a result, the cells are held snugly, without excessive physical stress, between the inside of a respective well and the surrounding gel cover.

As discussed hereinabove, there exist different types of gellable fluids including fluids that gel upon heating, fluids that gel upon cooling, fluids that gel upon irradiation or illumination, fluids that gel as a result of contact with a gelling reagent or fluids that gel after a period of time. In order to allow study of a cell held in a well according to the teachings of the present disclosure, it is generally preferred that the produced gel cover be transparent to the appropriate wavelength or wavelengths of light.

It is important to note that in general the teachings of the present disclosure are directed to the study of living cells. It is thus necessary that implementation of the method of the present disclosure not be lethal or toxic in any significant measure to living cells.

It is therefore preferred that a gel that is non-toxic and allows transport of molecules necessary for cell survival and for performing experiment is used for implementing the teachings of the present invention. Generally hydrogels are non-toxic and allows transport of molecules such as nutrients, gases, ions and waste to and from a living cell.

It is also preferred that a gellable fluid that gels under conditions that are conducive for cell survival be used for implementing the teachings of the present disclosure. One preferred gellable fluid is an alginate solution which gels upon contact with a solution having a Ca²⁺ ion concentration of greater than about 1 x 10⁻⁶ M, concentrations of Ca²⁺ ions that are suitable for cells. Another preferred gellable solution is a solution of low melting temperature agarose. Such solutions are fluid at relatively low temperature (*e.g*., 20°C) and gel at low temperatures that do not harm living cells (*e.g*., 4°C).

It is generally preferred that each well hold no more than one cell or no more than a predetermined number of cells. Most preferred is that the wells be of a size so as to accommodate no more than one cell. A suspension of cells with a number of cells greater than the number of wells is placed in proximity of the wells, and the cells allowed to settle. Excess cells that are "stacked" on top of cells held in wells are removed, before the gelling of the gellable fluid, for example by the application of a flow parallel to the top surface of a carrier of a device of the present invention, as described in PCT patent application IL01/000992. In such a manner, substantially all wells are populated and substantially no cells are left in the area of the matrix and not held in a well.

In some carriers of the present disclosure, there are few or no microfluidic features such as means for producing a flow parallel to the surface of the carrier. For example, in some embodiments of a carrier the present disclosure, substantially the only features are wells and interwell protuberances. In such cases, or for other reasons, it is not desired to or it is impossible to produce a flow of fluid parallel to the upper surface to wash away cells that are not held in wells. Therefore, an alternative method of loading a carrier of the present disclosure that is simpler but in certain instances may be considered inferior involves adding a suspension of cells in a gellable fluid (preferably a low temperature liquid agarose) in the proximity of the well matrix of a carrier of the present disclosure, where the approximate number of cells in the suspension is predetermined. The suspension with the carrier is centrifuged, driving the cells into the wells to be held therein. The gellable fluid is then gelled, for example, by cooling the the carrier during centrifugation. It has been found that when the number of cells in the suspension is approximately equal to the number of wells on the carrier, there is substantially one cell per well, with only minimal stacking of cells on top of already occupied wells.

In some embodiments of the method of the present disclosure where a gel cover is used with a well-bearing component, the wells of the well-bearing component are individually adressable. As discussed hereinabove when wells are individually adressable, it is simple to record and identify a cell or cells held in a specific well during the performance of an experiment. As a result, in such an embodiment a specific cell or cells can be identified and easily found, even subsequent to moving, transporting, shipping or storage with no fear that the motion will jostle the held cells out of the respective wells. For example, the well in which a cell having certain properties is held is noted. The well-bearing component is moved, for example, to be set in an incubator or sent to a different laboratory. The cell can thereafter be easily found by reference to the noted respective individually adressable well.

In some embodiments of the present invention, the bottoms of the wells are coplanar. When the bottoms of the wells are coplanar, the held cell or cells are easier to observe without the need for resorting to time-consuming refocussing. This is exceptionally true when each well holds only one cell. As a result, in such an embodiment the cells can repeatedly be examined, even subsequent to moving, transporting, shipping or storage with no fear that the motion will jostle the held cells out of the respective wells and necessitating time-consuming focussing.

In some embodiments of the present invention, the inside of the wells on which the cells rest is a proliferation-delaying surface. As discussed hereinabove, a proliferation delaying surface is a surface configured to suspend or reduce the rate of proliferation of cells in contact therewith.

One preferred type of proliferation delaying surface is an adhesion-delaying or inhibiting surface, that is the surface of the well on which the cell rests is coated with or made of a material with cell adhesion-delaying or inhibiting properties. As is known to one skilled in the art, many cell types proliferate only subsequent to adhesion to some surface or template. In embodiments of the present invention, either the inside of the wells are coated with an adhesion-delaying material or the well-bearing component (*e.g*., a carrier of the present invention) is substantially made of an adhesion-delaying or inhibiting material.

One class of adhesion-delaying materials includes polydimethylsiloxane. In accordance with the teachings of the present invention, a polydimethylsiloxane adhesion-delaying material can include polydimethylsiloxane as one adhesion-delaying component, can be substantially of polydimethylsiloxane or can be substantially of pure polydimethylsiloxane. One commercially available polydimethylsiloxane that has been found to be useful for implementing the teachings of the present invention is RTV 615 (GE Silicones, Wilton, CT, USA).

Another preferred type of proliferation delaying surface is a gel, that is the surface of the well on which the cell rests is coated with or made of a gel that has proliferation-delaying properties. In embodiments of the present disclosure, either the inside of the wells are coated with a layer of gel or the well-bearing device (*e.g*., a. carrier of the present invention) is substantially made of a gel, preferably a hydrogel. It is important to note that the gel that is the surface of the wells can be the same, similar or different as the gel from which the gel cover is made. In such an embodiment, subsequent to the formation of a gel cover as described hereinabove, the cells held in wells are in fact encased inside gel, each cell in a respective gel pocket. One commercially available gel that has been found to be useful for implementing the teachings of the present disclosure is a sodium alginate solution marketed under the name Protanal LF120 1% in water (Pronova Biopolymers, Drammen, Norway).

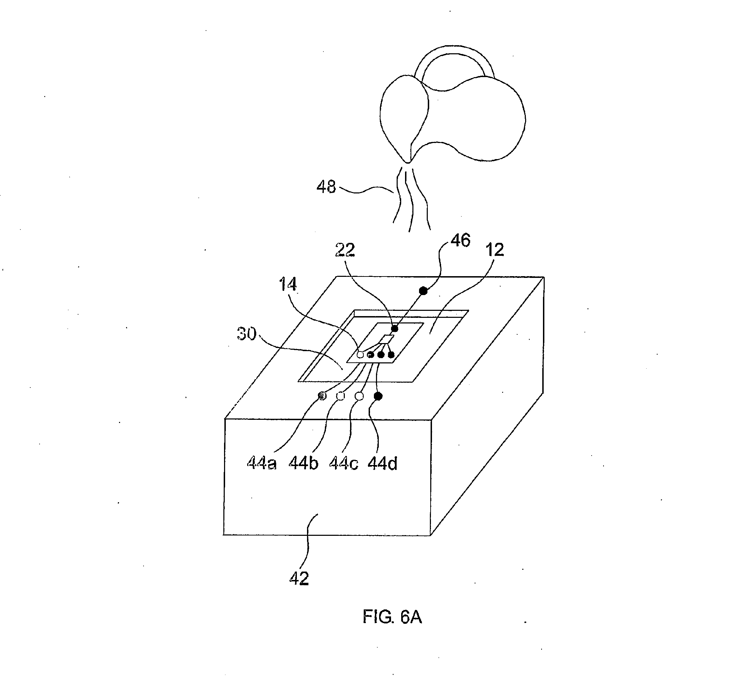

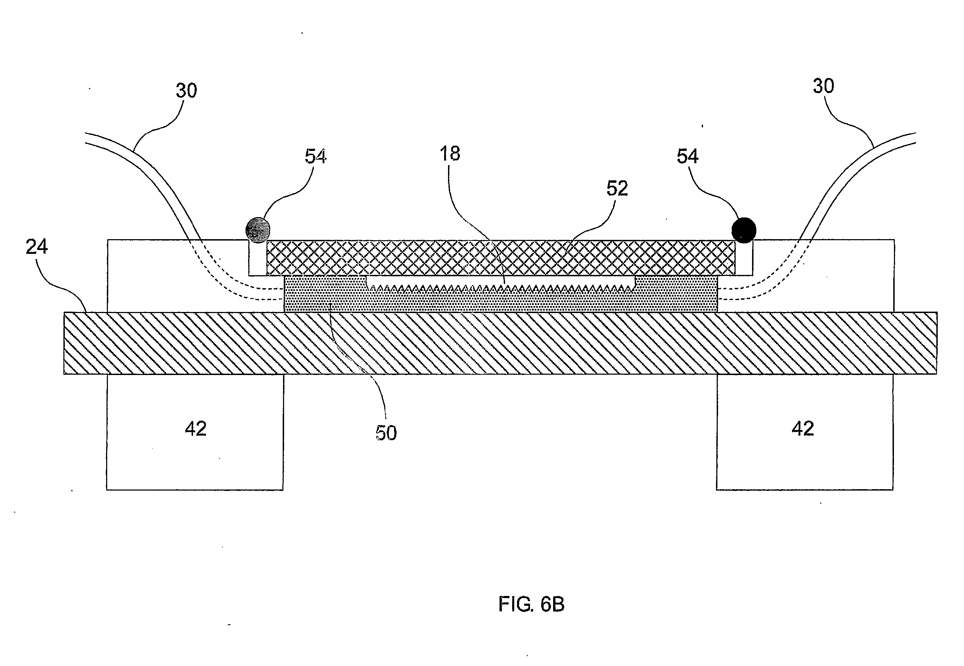

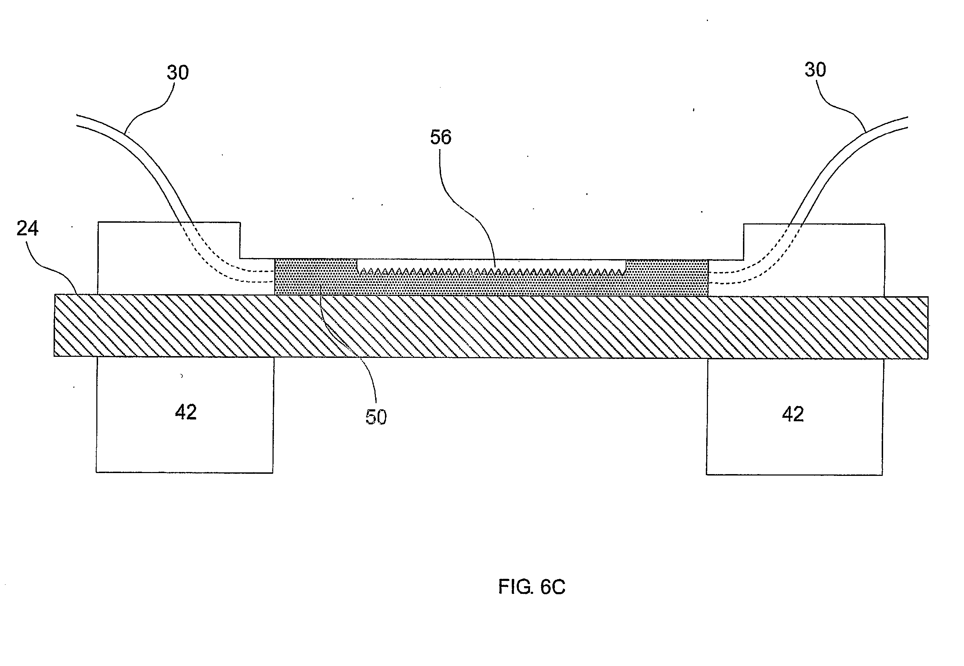

The use of a gel cover together with a gel adhesion-delaying carrier is discussed with reference to Figures 6A, 6B and 6C. A glass carrier **12** as described in PCT patent application IL01/00992 including a matrix of hexagonally packed wells, four input ports and one output port, substantially as described in Figure 1 is provided and held in place in rubber frame **42**, Figure 6A. Apart from serving to hold glass carrier **12**, rubber frame **42** is provided with four input ports **44a**, **44b**, **44c** and **44d**, and an output port **46** which are in communication with the inlet connectors **14** and outlet connector **22**, respectively by capillary tubing **30**. A rubber polymer **48** (*e.g*., a hydrophilic vinyl polysiloxane impression material, available as Examix® NDS from GC America Inc., Alsip, IL, USA) is poured into rubber frame **42** and allowed to harden forming a rubber negative mold of glass carrier **12**. Subsequent to hardening, the rubber negative mold is removed from rubber frame **42**, and glass carrier **12** removed from rubber frame **42**. A gellable fluid (molten agar at 70 °C) is poured into rubber frame **42**, and the rubber negative mold put in place in rubber frame **42**. After a few hours, the gellable fluid has gelled and cooled forming a gel carrier. The rubber negative mold is removed and a device assembled using the newly formed gel carrier.

In Figure 6B, depicted in side cross-section is a gel carrier **50** resting on a transparent holder **24** and held in place by rubber frame **42**. A cover slip **52** is held in place above gel carrier **50** by a gasket **54**. In such a way, gel carrier **50** and cover slip **52**, supported by rubber frame **42** define a sealed volume including a matrix of wells **18**, and fluid flow passages in communication with four flow-generating devices attached to respective input ports **44a**, **44b**, **44c** and **44d** through capillary tubing **30** (represented in Figure 6B as dashed lines. A suspension of cells in a physiological fluid having a Ca²⁺ ) concentration of 1mM is injected through input port **44a** and is transported through capillary tubing **30** to the vicinity of matrix of wells **18.** The suspended, cells are allowed to settle into individual wells of matrix of wells **18.** Excess cells that have not settled into a well and physiological fluid are washed away by application of a Ca²⁺-free buffer solution in a flow parallel to the surface of carrier introduced through input port **44b.** A gellable fluid (an alginate) containing calcium gluconate is injected through input port **44c.** The concentration of calcium gluconate in the gellable fluid is such that the onset of gelling takes about 30 minutes after the fluid is injected. After about 30 minutes, the gellable fluid gels, trapping cells inside a proliferation delaying sandwich.

In Figure 6C, depicted in side cross-section is a gel carrier **50** resting on a transparent holder **24** and held in place by rubber frame **42,** where above gel carrier **50** is a gel cover **56** of the present disclosure.

An exceptionally useful experimental method that is advantageously performed using the teachings of the present disclosure involves physically isolating a cell. Once cells are snugly held in wells under a gel cover, individual cell (or cells) is isolated by excising the cell (or cells) from the well-bearing component. The cell or cells can be further manipulated, encased within the gel, or released by dissolution of the gel. Clearly excision of cells is most easily accomplished when the well-bearing component is a carrier made of a gel.

It is important to note, that when isolating a cell or cells as described immediately hereinabove, it is exceptionally useful that the wells be individually addressable.

In a typical experiment implementing the experimental method described immediately hereinabove, a carrier with a plurality of fluorescent fiducial points is fashioned from a gel so as to render the wells individually addressable. A cell-containing fluid is brought in proximity of the wells of the carrier so that cells in the fluid settle into wells of the carrier to be held therein. A flow of fluid is applied parallel to the surface of the carrier to wash away cells that are not held in wells. Subsequently, a gellable fluid is added and gelled. The snugly held cells inside the gel covered gel carrier are transferred to a microscope and examined. The wells holding cells having a specific property are noted in reference to the fluorescent fiducial points. Subsequently, the gel covered gel carrier is transferred to a cutting machine where the cells having the specific property are excised and separated from the cells not having the specific property.

An additional useful embodiment of the method of the present disclosure includes the addition of active entities with or through the gel cover (or, if applicable, the gel carrier). In a first embodiment, active entities are added subsequent to gelling by contacting an active entity (generally in solution) with the surface of the gel (be it a gel cover or a gel carrier). Over the following period of time the active entity diffuses into and through the gel to the vicinity of the cells held snugly underneath the gel cover. Advantages of this first embodiment include addition of active entities only when needed. In a second embodiment, active entities are mixed with the gellable fluid before gelling. Advantages of the second embodiment include that large active entities that diffuse through a gel only with difficulty or not at all can be trapped within the gel matrix and that the active entities are homogenously distributed throughout the gel. Typical active entities useful in implementing the teachings of the present invention are listed hereinabove.

In a typical experiment implementing the experimental method described immediately hereinabove, a carrier is fashioned from a gel containing a chromatogenic reagent sensitive to a waste product related to cell metabolism or other secreted compound (*e.g*., antibodies, enzymes and the like) as a first active entity. A cell-containing fluid is added above the wells so that cells in the sample settle into wells of the carrier to be held therein. A flow of fluid is applied parallel to the carrier to wash away cells that are not held in wells. Subsequently, a gellable fluid containing a selective toxin configured to kill cells having a specific mutation as a second active entity is added and gelled. The gel-covered gel carrier is ) bathed in a nutrient solution and the development of color by the first active entity is monitored. Cells having the specific mutation are killed by the action of the second active entity and do not generate a color. In contrast, cells not having the specific mutation are easily identified by the generated color.

It is important to note an added advantage of the prevent disclosure. Since a gel matrix reduces the rate of diffusion of compounds secreted from the cell as compared to regular physiological media and as there is little or no fluid flow inside the gel, it becomes possible, using the teachings of the present disclosure, to clearly identify which cell secretes a given compound, contingent on the existence of an appropriate indicator or detection method.

As discussed hereinabove, a problem in the art is that of proliferation of cells held or isolated in well-bearing devices. A cell is held in a well. If the well is uncovered, movement of the well-bearing component causes cells to move out from a well, either being lost or losing identity. Even if the well-bearing component is not moved, proliferation of cells inside an enclosure leads to unnatural population shapes, cell distortion and overcrowding effects. Further, if the cell populations grow outwards from the well, the cells are subject to flow induced loss or migration from the population itself. The teachings of the present disclosure provide a number of solutions for these problems.

An useful embodiment of the method of the present disclosure includes allowing cells snugly held under a gel cover to proliferate into or through the gel cover (or, if applicable, the gel carrier).

In a typical experiment implementing the experimental method described immediately hereinabove, a carrier having coplanar wells is fashioned from a gel. A cell-containing fluid is added in proximity of the wells so that cells in the sample settle into wells of the carrier to be held therein. A flow of fluid is applied parallel to the carrier to wash away cells that are not held in wells. Subsequently, a gellable fluid containing a chromatogenic active entity is added, the chromatogenic active entity configured to generate a color only when in contact with a specific cell-surface receptor. The gel-covered gel carrier is automatically interrogated with image-processing software using a computer-controlled camera. Cells held in wells where color is not generated are destroyed by irradiation with a laser. After all mutation-less cells are destroyed the gel-covered gel carrier is transported to a remote laboratory. The living cells remain snugly held inside a gel matrix and are not lost. The proliferation delaying properties of the gel ensure that during transport no additional cells develop. When arriving at the remote laboratory, the cells are allowed to proliferate under observation. Since the cells are snugly held, the cells remain coplanar allowing quick and efficient automatized observation. The cells proliferate into and through the gel and do not suffer from the effects of overcrowding or limited volume.

Another solution for the problems related to the proliferation of cells held in prior art a well-bearing device, such as a chip-device, is by holding at least one cell in a well of a well-bearing device and subsequently increasing the size of the well so as to provide an increased space for proliferation of the cell. Clearly, a preferred device is a carrier of a chip-device having changeable well sizes, as described hereinabove. Generally, a plurality of cells is held in wells of the well-bearing device. The cells are examined (*e.g*., through visual interrogation, chemical/biological reaction using an active entity, or a combination of active entities) and certain cells selected. The non-selected cells are discarded (*e.g*., by physical removal, for example using optical tweezers or by damaging (*e.g*., killing) the non-selected cells). Once the non-selected cells are discarded, the cell size is increased giving sufficient room for the cells to proliferate without problems discussed above.

In one embodiment of the present invention a carrier having a changeable well-size is elastically deformable, for example, the carrier is made substantially of an elastically deformable material including but not limited to elastomers, rubber, silicon rubbers or other materials, for example as listed in U.S. Patent 6,740,727, U.S. Patent 6,682,792 and U.S. Patent 6,673,857. Such carriers are placed in a deforming device and by the application of tension stretched to a desired extent. The elastically deformable carrier remains in a deforming device and the tension maintained for as long as the changed size is desired.

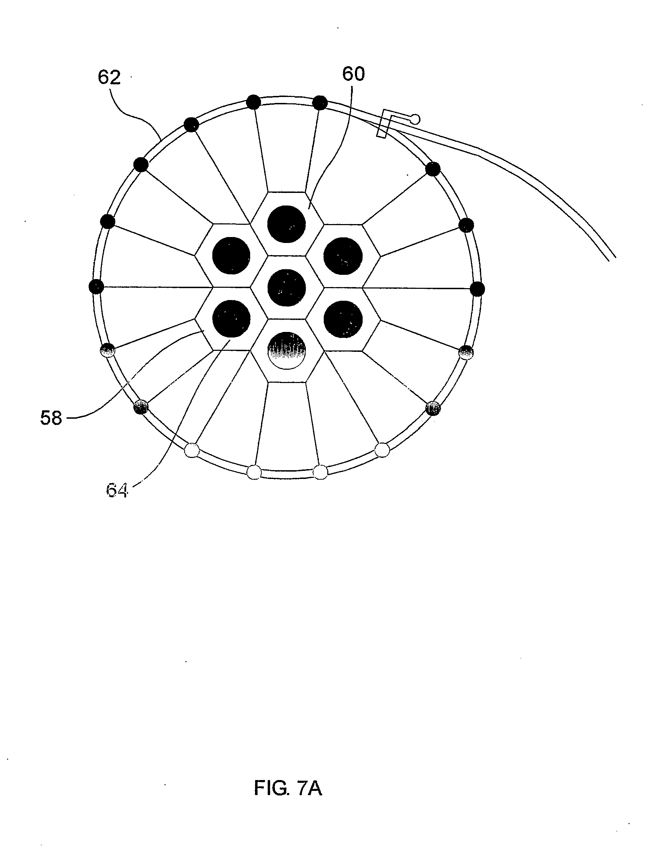

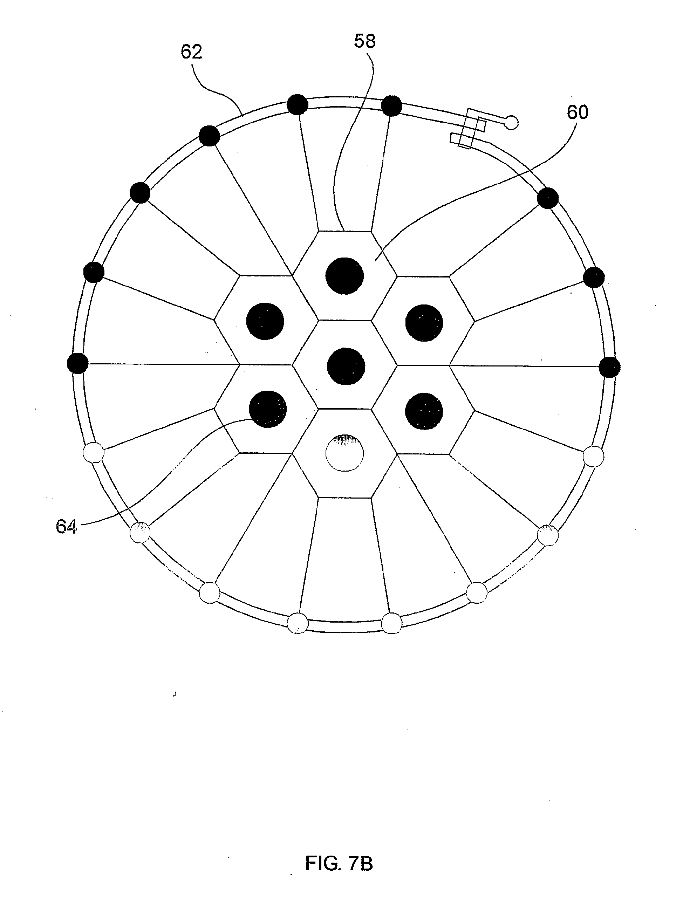

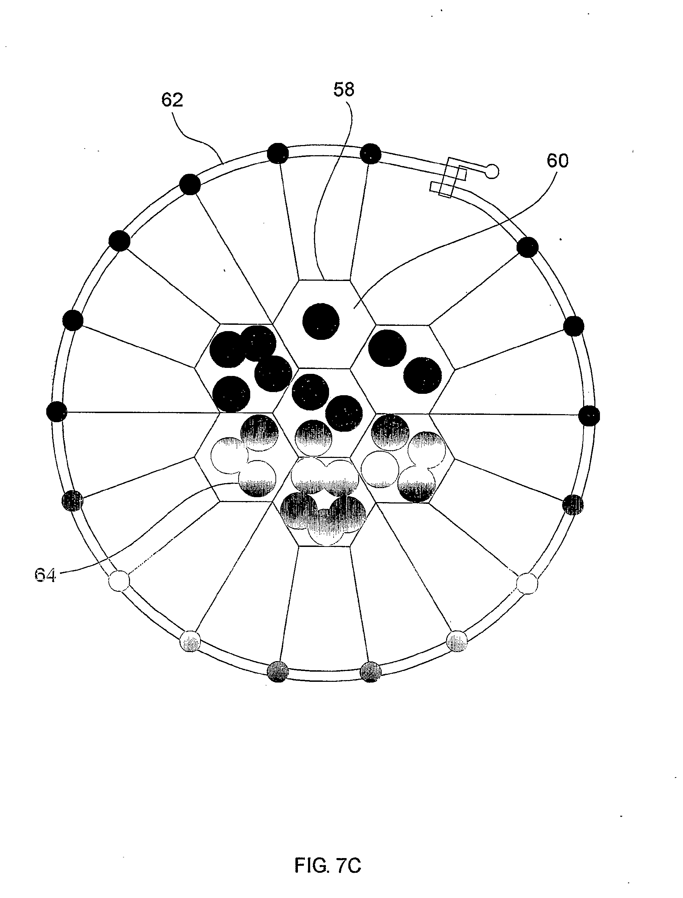

The use of an elastically deformable carrier is discussed with reference to Figures 7A, 7B and 7C. An elastically deformable carrier **58** made of a silicon rubber having a 700% elongation range (*e.g*., Silastic® LSR 9280-30, Dow Corning Corporation, Midland, MI, USA) with a matrix of hexagonally packed hexagonal enclosures **60** is fabricated by press-molding followed by heat curing. Each enclosure **60** has a diameter and a depth of 20 micron. Elastic carrier **58** is placed in a holder / stretching device **62**, Figure 7A. Cells **64** in a cell-containing suspension are allowed to settle into and be held in enclosures **60**. Stretching device **62** is used to stretch elastic carrier **58** so that each of enclosure **60** has a diameter of 100 micron, Figure 7B. Additionally, a gellable fluid may be applied over the expanded enclosures and gelled as described above, forming a gel cover that prevents cells **64** held in enclosures **60** from being lost. Carrier **58**, together with the gel cover and stretching device **62** may be moved to an incubator. After some time, cells **64** held in enclosures **60** have proliferated, Figure 7C.

In another embodiment of the present invention a carrier having a changeable well-size is plastically deformable, for example, the carrier is made substantially of a hydrocarbon wax. By plastically deformable material is meant a material that does not recover shape after deformation. Such carriers are placed in a deforming device and by the application of tension stretched to a desired extent. Tension can be released as the carrier does not recover to the former shape.

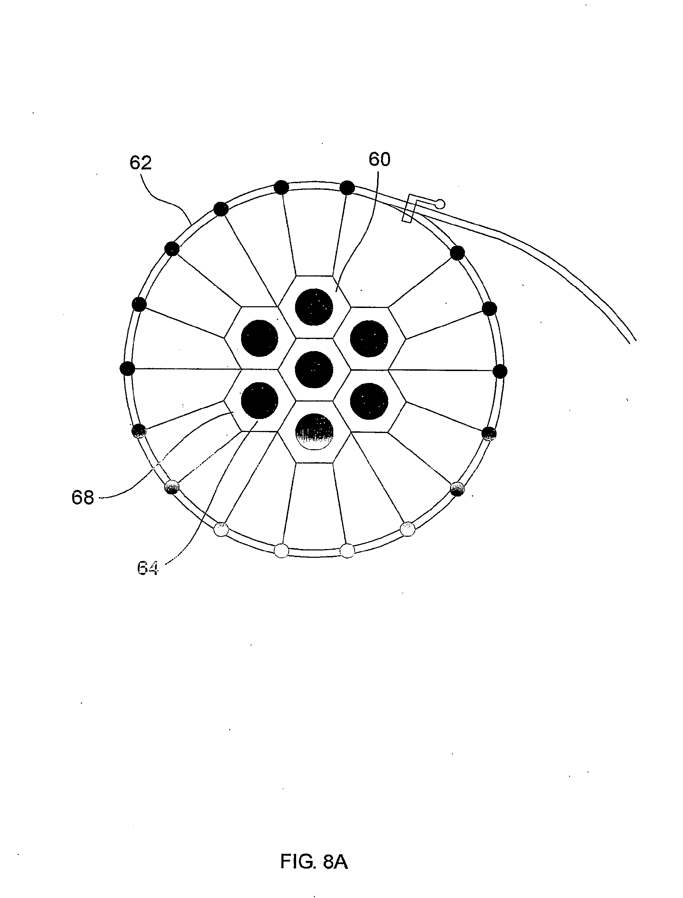

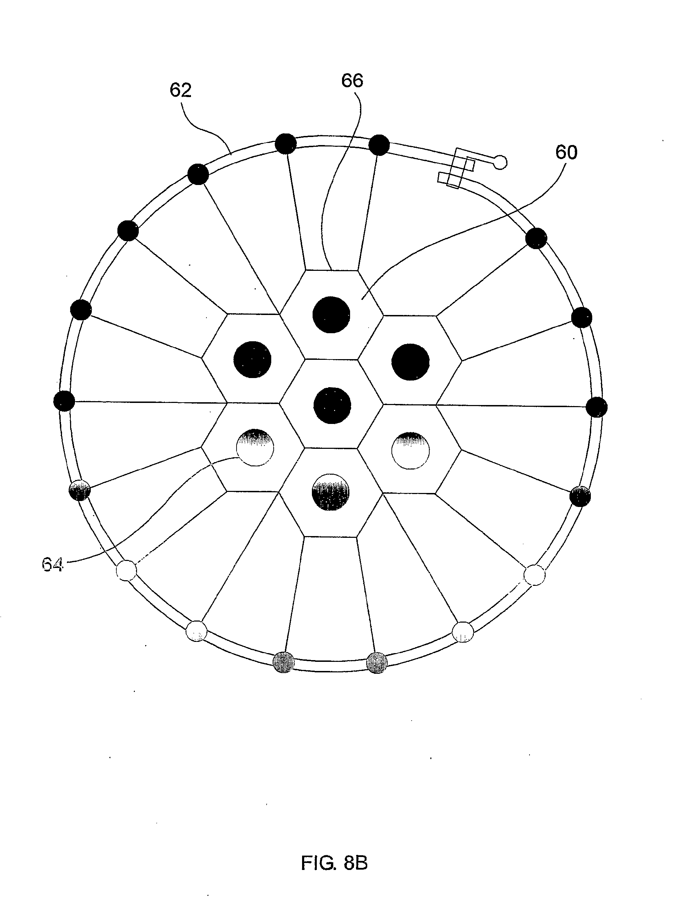

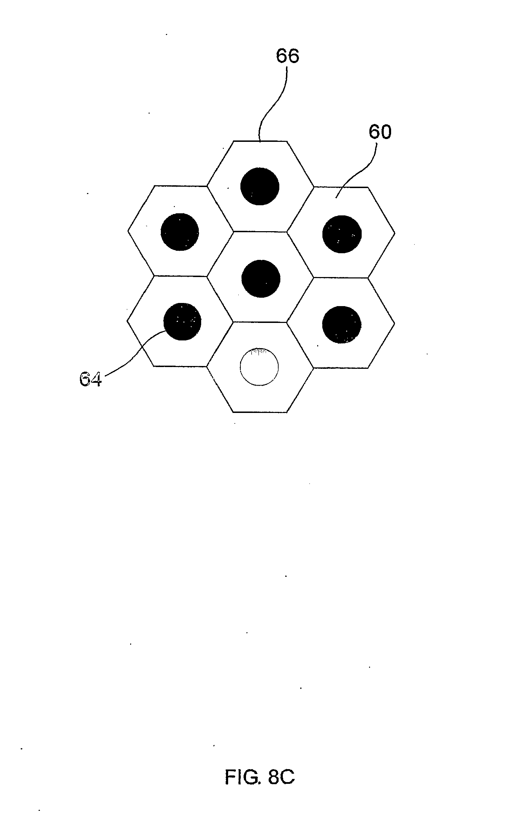

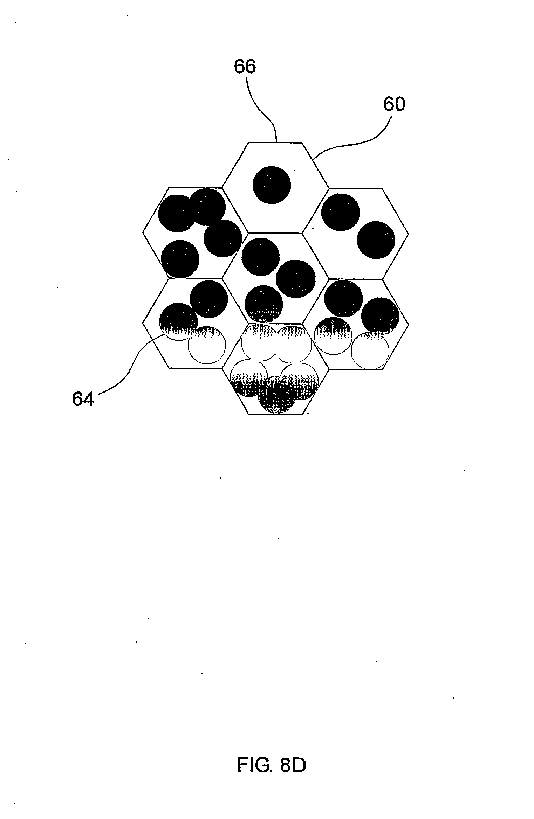

The use of a plastically deformable carrier is discussed with reference to Figures 8A, 8B, 8C and 8D. A sheet of hydrocarbon wax (PARAFILM®, Pechiney Plastic Packaging, Inc., Neenah, WI, USA) is placed in a stretching device and pulled to be flat but with no plastic deformation. A stamp is used to apply a pattern to the surface of the wax sheet so as to make a matrix of hexagonally packed hexagonal enclosures.

Each enclosure **60** has a diameter and a depth of 20 micron. Plastically deformable carrier **66** is placed in a holder / stretching device **62**, Figure 8A. Cells **64** in a cell-containing suspension are allowed to settle into and be held in enclosures **60**. Stretching device **62** is used to stretch Plastically deformable carrier **66** so that each of enclosure **60** has a diameter of 100 micron, Figure 8B. According to one embodiment, a gellable fluid may be applied over the expanded enclosures and gelled as described above, forming a gel cover that prevents cells **64** held in enclosures **60** from being lost. Plastically deformable carrier **66** is released from stretching device **62** and, together with the gel cover, is moved to an incubator, Figure 8C. After some time, cells **64** held in enclosures **60** have proliferated, Figure 8D. When sufficient proliferation occurs, cells are harvested from each clone for further examination, with the possibility of leaving at least one cell in the original enclosure.

In a variation of the methods discussed immediately hereinabove, a series of expandable carriers is provided. The size of wells of one carrier of the series in the expanded state is substantially similar to the size of wells on the succeeding carrier of the series in the non-expanded state. In such a way, a cell is isolated in a well of a first carrier. The size of the well of the first carrier is expanded. The succeeding carrier is laid on top of the first carrier. The two carriers are inverted so that cells in the expanded wells of the first carrier drop into the the non-expanded wells of the second carrier. The process is repeated until the cell is deemed to have sufficient space to proliferate.

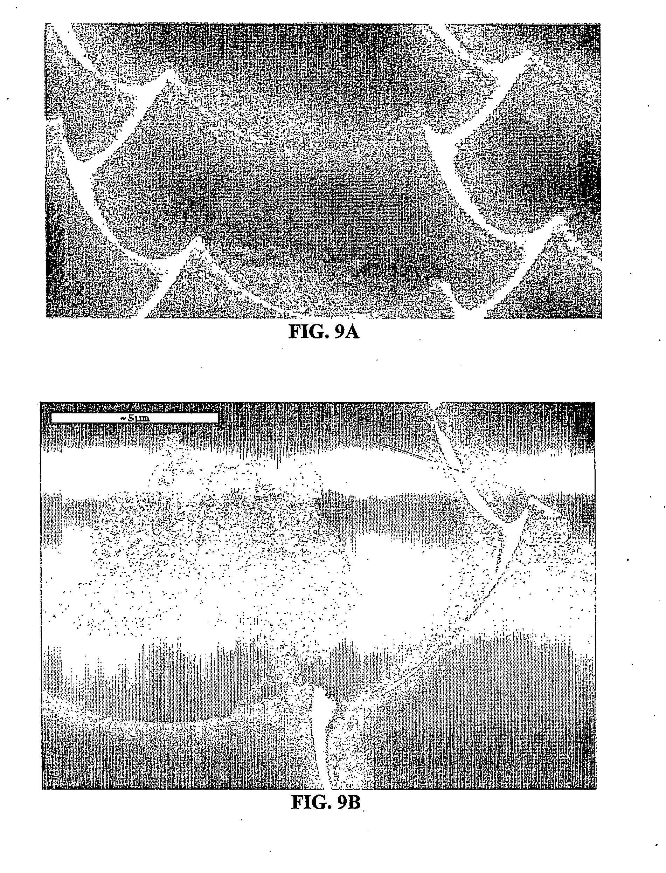

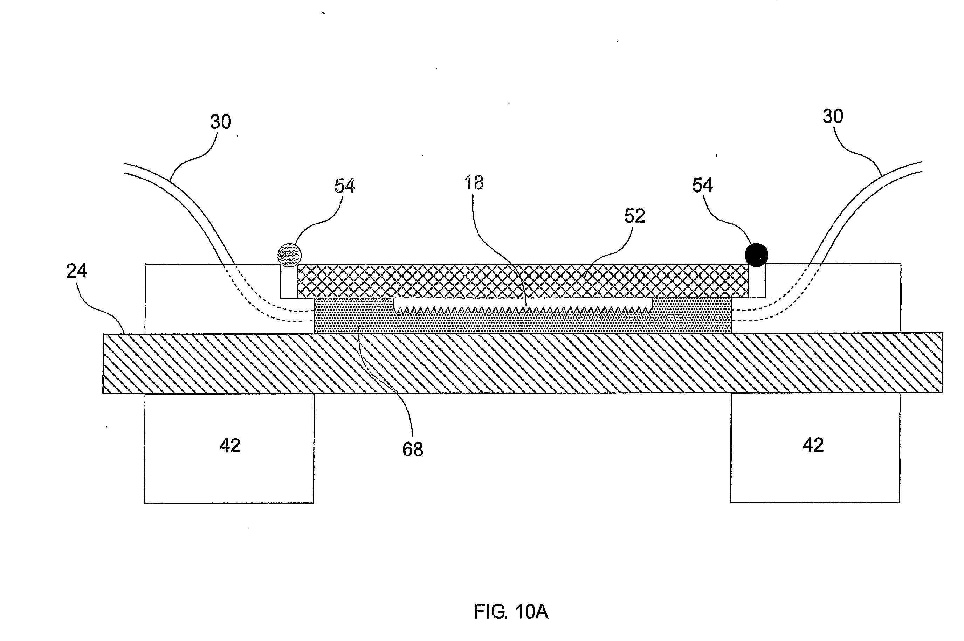

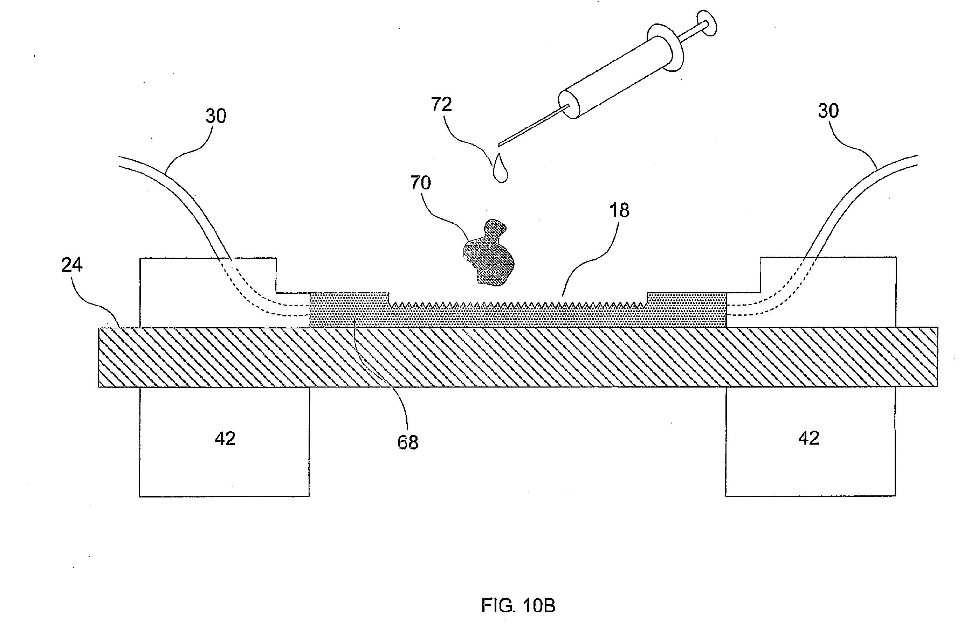

An typical procedure for collecting cells from a tumor is described with reference to Figures 10A and 10B. A transparent carrier **68**, made in accordance with the teachings of the present invention or in accordance with the teachings of PCT patent application IL01/000992, is provided having a well matrix **18** including a matrix of hexagonally packed knife-edged hexagonal wells having a plurality of protuberances emerging from the area between any three wells, similar to the protuberances depicted in Figures 9A and 9B. Transparent carrier **68** is mounted in a holder **42**, where cover slip **52** is held in place by gasket **54**. An inlet flow device is in comunication with the fluid channels of transparent carrier **69** through capillary tubes **30**. The inlet flow device is activated, filling the fluid channels, reservoirs and wells with fluid and driving air bubbles out of the system, Figure 10A.

Once the fluid channels of carrier **68** are filled with fluid and devoid of air, gasket **54** is released and cover slip **52** removed, exposing well matrix **18**. A biological sample **70**, (*e.g*., a tumor) is pressed against well matrix **18** while fluid **72** is made to flow along biological sample **70**, Figure 10B. After a few seconds or up to a few minutes, during which time cells freed from biological sample **70** by the action of the protuberances of well matrix **18** settle down into wells, biological sample **70** is set aside, cover slip **52** and gasket **54** returned to the proper position and study of the thus-collected cells performed in the usual way.

As is clear to one skilled in the art, the quick and simple method of harvesting cells of the present disclosure allows, for example, high throughput and efficient screeing of biological samples, for example in the fields of genetics, diagnostics and oncology.

In a typical example, a transparent polydimethylsiloxane carrier with a surface having hexagonally packed individually addressable wells with protuberances surrounded by a wall is placed in chip-device. A drop of physiological fluid is placed on the carrier so as to completely fill the wells with the fluid. A tumor is excised from a patient and pressed against the surface while more physiological fluid is dripped along the tumor. A glass cover slip with cover slip electrodes is placed on top of the carrier so as to rest on the wall of the carrier. As described in PCT patent application IL01/000992, individual cells are held in individual respective wells. A chromatogenic reagent configured to generate a color upon contact with a pathological cell is introduced. Cells that do not react with the reagent are extracted by the application of an attractive force from a cover slip electrode associated with the respective well. A diagnosis is then made based on the reaction with the reagent. When only pathological cells remain in the well, a gellable fluid is introduced and gelled, trapping the held cells between the dimethylsiloxane carrier and a gel cover. The carrier and trapped cells are then stored in non-proliferating conditions for further examination and analysis if required.

Additional objects, advantages, and novel features of the present disclosure will become apparent to one ordinarily skilled in the art upon examination of the following examples, which are not intended to be limiting.

### EXPERIMENTAL RESULTS

### Cell-Proliferation delay of polydimethylsiloxane (PDMS)

A standard glass petri dish (Nunc S/A, Roskilde, Denmark) and two petri dishes made of polydimethylsiloxane, Dish 1 using RTV615 PDMS (GE Silicones, Wilton, CT, USA) and Dish 2 using Sylgard 184 PDMS (Dow Coming Corporation, Midland, MI, USA) were provided.

Thawed frozen PC3 prostate cancer cells (DSMZ GmbH, Braunschweig, Germany) were cultured at 37°C in RPMI medium with 10% Fetal Calf Serum in each of the three dishes. The development on the cells was observed for four days (Table 1A). After four days, the cells were relocated to identical dishes under identical conditions and again cell development was observed for four days (Table 1B).

**Table 1A: PC3 cell proliferation in glass and PDMS dishes, Days 0-4**

| Incubation | 24 h | 48 h | 72 h | 96h |
|---|---|---|---|---|
| Glass | Adherence | proliferation start | proliferation | proliferation |
| Dish 1 | no adherence | adherence | proliferation start | proliferation |
| Dish 2 | no adherence | adherence | proliferation start | proliferation |

**Table 1B: PC3 cell proliferation in glass and PDMS dishes, Days 5-8**

| | | | | |
|---|---|---|---|---|
| Total time | 120 h | 144 h | 168 h | 192 h |
| Incubation | 24 h | 48 h | 72 h | 96h |
| Glass | Adherence | proliferation start | proliferation | proliferation |
| Dish 1 | no adherence | no adherence | adherence | proliferation start |
| Dish 2 | no adherence | no adherence | adherence | proliferation start |

From the results observed and summarized in Tables 1A and 1B, it is seen that polydimethylsiloxane delays cell adherence to a surface and thus delays-proliferation. It is important to note that the non-cytotoxicity of cells of polydimethylsiloxane was confirmed as no cell deaths were observed.

Generally, the nomenclature used herein and the laboratory procedures utilized in the present invention include techniques from the fields of biology, chemistry and engineering. Such techniques are thoroughly explained in the literature.

In addition, citation or identification of any reference in this application shall not be construed as an admission that such reference is available as prior art to the present invention.

## Claims

1. A chip-device for holding living cells, the device comprising:
a carrier having a plurality of wells disposed on a surface thereof with each well configured to hold at least one and no more than one living cell,
wherein the carrier is formed of an elastically or plastically deformable material; and
a deforming device in which the carrier is mounted to apply tension to expand the carrier to enlarge the size of the wells and provide increased space for cell proliferation, thereby to influence the proliferation of living cells held in the wells.

2. The device of claim 1, wherein the wells are formed by or coated with a material that influences cell proliferation by at least one of:
(a) delaying cell proliferation;
(b) inhibiting cell proliferation;
(c) delaying adhesion of living cells to the insides of the wells;
(d) inhibiting adhesion of living cells to the inside of the wells.

3. A device according to claim 1 or claim 2, wherein the individual wells in the unexpanded state have a diameter and a depth of 20 microns, and a diameter in the expanded state of 100 microns.

4. A device according to claim 1 or claim 2, wherein the size of the individual wells is in the range of 200 microns to 10 microns

5. A method of making a chip-device, wherein the chip device comprises a carrier having plurality of wells disposed on a surface thereof with each well configured to hold at least one and no more than one living cell, wherein the carrier is formed of an elastically or plastically deformable material and wherein the carrier and the wells are expandable and thereby influence the proliferation of living cells held in the wells, the method comprising:
(a) providing a template having a negative of the features of the wells and the carrier;
(b) contacting the template with a precursor material so as to create the features in the precursor material;
(c) fixing the features in the precursor material to fashion the carrier; and
(d) placing the carrier in a holder/stretching device operable to stretch the carrier to expand the diameter of the wells from 20 microns to 100 microns,

6. The method of claim 5, wherein the precursor material is plastically deformable and the features are fixed by separating the template from the precursor material.

7. The method of claim 5, wherein the precursor material is elastically deformable,

8. A method of growing cells comprising:
(a) providing a well-bearing device wherein the well-bearing device is comprised of::
a carrier having a plurality of wells disposed on a surface thereof wherein the carrier is formed of an elastically or plastically deformable material; and a deforming device in which the carrier is mounted to apply tension to expand the carrier to enlarge the size of the wells and provide increased space for cell proliferation, thereby to influence the proliferation of living cells held in the wells;
(b) holding at least one and no more than one living cell in a well of the chip device; and
(c) influencing proliferation of the cells by application of tension to the carrier to increase the size of the wells thereby to provide increased space for proliferation.

9. The method according to claim 8, wherein the carrier and the wells are formed of an elastically deformable material, and the tension on the carrier is maintained after the desired increase in size.

10. The method according to claim 8, wherein the carrier and the wells are formed of a plastically deformable material, and the tension on the carrier is released after the desired increase in size.

## Patentansprüche

1. Chipvorrichtung zum Aufnehmen von lebenden Zellen, wobei die Vorrichtung umfasst:
einen Träger mit einer Mehrzahl von Wannen, die auf einer Oberfläche davon angeordnet sind, wobei jede Wanne so ausgelegt ist, dass sie mindestens eine und nicht mehr als eine lebende Zelle aufnimmt,
wobei der Träger aus einem elastisch oder plastisch verformbaren Material gebildet ist; und
eine Verformungsvorrichtung, in welcher der Träger montiert ist, um Spannung aufzubringen, um den Träger zu dehnen, um die Größe der Wannen zu vergrößern und einen größeren Raum zur Zellproliferation bereitzustellen, um dadurch die Proliferation von lebenden Zellen zu beeinflussen, die in den Wannen aufgenommen sind.

2. Vorrichtung nach Anspruch 1, wobei die Wannen durch ein Material gebildet oder mit einem Material beschichtet sind, welches Zellproliferation durch mindestens eines von Folgendem beeinflusst:
(a) Verzögern von Zellproliferation;
(b) Hemmen von Zellproliferation;
(c) Verzögern von Haftung von lebenden Zellen an den Innenseiten der Wannen;
(d) Hemmen von Haftung von lebenden Zellen an den Innenseiten der Wannen;

3. Vorrichtung nach Anspruch 1 oder 2, wobei die einzelnen Wannen im nicht gedehnten Zustand einen Durchmesser und eine Tiefe von 20 Mikrometern und im gedehnten Zustand einen Durchmesser von 100 Mikrometern aufweisen.

4. Vorrichtung nach Anspruch 1 oder 2, wobei die Größe der einzelnen Wannen im Bereich von 200 Mikrometern bis 10 Mikrometern liegt.

5. Verfahren zur Herstellung einer Chipvorrichtung, wobei die Chipvorrichtung einen Träger mit einer Mehrzahl von Wannen umfasst, die auf einer Oberfläche davon angeordnet sind, wobei jede Wanne so ausgelegt ist, dass sie mindestens eine und nicht mehr als eine lebende Zelle aufnimmt, wobei der Träger aus einem elastisch oder plastisch verformbaren Material gebildet ist, und wobei der Träger und die Wannen dehnbar sind und dadurch die Proliferation von lebenden Zellen beeinflussen, die in den Wannen aufgenommen sind, wobei das Verfahren umfasst:
(a) Bereitstellen einer Schablone mit einem Negativ der Merkmale der Wannen und des Trägers;
(b) In-Kontakt-bringen der Schablone mit einem Vorläufermaterial, um die Merkmale im Vorläufermaterial zu bilden;
(c) Fixieren der Merkmale im Vorläufermaterial, um den Träger herzustellen; und
(d) Anordnen des Trägers in einer Halte-/Streckvorrichtung, die so betrieben werden kann, dass sie den Träger streckt, um den Durchmesser der Wannen von 20 Mikrometern auf 100 Mikrometer auszudehnen.

6. Verfahren nach Anspruch 5, wobei das Vorläufermaterial plastisch verformbar ist und die Merkmale durch Trennen der Schablone vom Vorläufermaterial fixiert werden.

7. Verfahren nach Anspruch 5, wobei der das Vorläufermaterial elastisch verformbar ist.

8. Verfahren zur Züchtung von Zellen, umfassend:
(a) Bereitstellen einer wannentragenden Vorrichtung, wobei die wannentragende Vorrichtung umfasst:
einen Träger mit einer Mehrzahl von Wannen, die auf einer Oberfläche davon angeordnet sind, wobei der Träger aus einem elastisch oder plastisch verformbaren Material gebildet ist; und eine Verformungsvorrichtung, in welcher der Träger montiert ist, um Spannung aufzubringen, um den Träger zu dehnen, um die Größe der Wannen zu vergrößern und einen größeren Raum zur Zellproliferation bereitzustellen, um dadurch die Proliferation von lebenden Zellen zu beeinflussen, die in den Wannen aufgenommen sind;
(b) Aufnehmen mindestens einer und nicht mehr als einer lebenden Zelle in einer Wanne der Chipvorrichtung; und
(c) Beeinflussen der Proliferation der Zellen durch Aufbringen von Spannung auf den Träger, um die Größe der Wannen zu vergrößern und einen größeren Raum zur Proliferation bereitzustellen.

9. Verfahren nach Anspruch 8, wobei der Träger und die Wannen aus einem elastisch verformbaren Material gebildet sind, und die Spannung auf den Träger nach der gewünschten Vergrößerung der Größe aufrechterhalten wird.

10. Verfahren nach Anspruch 8, wobei der Träger und die Wannen aus einem plastisch verformbaren Material gebildet sind, und die Spannung auf den Träger nach der gewünschten Vergrößerung der Größe gelöst wird.

## Revendications

1. Dispositif de type puce destiné à contenir des cellules vivantes, le dispositif comprenant :
un support ayant une pluralité de puits disposés sur une surface de celui-ci, chaque puits étant configuré pour contenir au moins une et pas plus d'une seule cellule vivante,
où le support est formé d'un matériau capable de subir une déformation élastique ou plastique ; et
un dispositif de déformation dans lequel le support est monté pour appliquer une tension et étendre le support afin d'agrandir la taille des puits et d'obtenir un espace accru pour la prolifération cellulaire, afin d'ainsi influencer la prolifération des cellules vivantes contenues dans les puits.

2. Dispositif selon la revendication 1, dans lequel les puits sont formés par ou sont revêtus d'un matériau qui influence la prolifération cellulaire par au moins l'un parmi :
(a) un retard de la prolifération cellulaire ;
(b) une inhibition de la prolifération cellulaire ;
(c) un retard de l'adhésion des cellules vivantes à l'intérieur des puits ;
(d) une inhibition de l'adhésion des cellules vivantes à l'intérieur des puits.

3. Dispositif selon la revendication 1 ou la revendication 2, dans lequel les puits individuels dans l'état non étendu possèdent un diamètre et une profondeur de 20 microns, et un diamètre dans l'état étendu de 100 microns.

4. Dispositif selon la revendication 1 ou la revendication 2, dans lequel la taille des puits individuels est comprise dans la plage de 200 microns à 10 microns.

5. Procédé de fabrication d'un dispositif de type puce, où le dispositif de type puce comprend un support ayant une pluralité de puits disposés sur une surface de celui-ci, chaque puits étant configuré pour contenir au moins une et pas plus d'une seule cellule vivante, où le support est formé d'un matériau capable de subir une déformation élastique ou plastique et où le support et les puits peuvent être étendus et ainsi influencer la prolifération des cellules vivantes contenues dans les puits, le procédé comprenant :
(a) la mise à disposition d'une matrice comportant un négatif des caractéristiques des puits et du support ;
(b) la mise en contact de la matrice avec un matériau précurseur de sorte à créer les caractéristiques dans le matériau précurseur ;
(c) la fixation des caractéristiques dans le matériau précurseur afin de façonner le support ; et
(d) la mise en place du support dans un dispositif de maintien/d'élongation fonctionnel pour étirer le support et augmenter le diamètre des puits de 20 microns à 100 microns.

6. Procédé selon la revendication 5, dans lequel le matériau précurseur est capable de subir une déformation plastique et les caractéristiques sont fixées en séparant la matrice du matériau précurseur.

7. Procédé selon la revendication 5, dans lequel le matériau précurseur est capable de subir une déformation élastique.

8. Procédé de culture de cellules, comprenant :
(a) la mise à disposition d'un dispositif comportant des puits, où le dispositif comportant des puits est composé de :
un support ayant une pluralité de puits disposés sur une surface de celui-ci, où le support est formé d'un matériau capable de subir une déformation élastique ou plastique ; et un dispositif de déformation dans lequel le support est monté pour appliquer une tension et étendre le support afin d'agrandir la taille des puits et d'obtenir un espace accru pour la prolifération cellulaire, afin d'ainsi influencer la prolifération des cellules vivantes contenues dans les puits ;
(b) le maintien d'au moins une et pas plus d'une seule cellule vivante dans un puits du dispositif de type puce ; et
(c) l'influence de la prolifération des cellules par l'application d'une tension au support afin d'augmenter la taille des puits pour ainsi obtenir une espace accru pour la prolifération.

9. Procédé selon la revendication 8, dans lequel le support et les puits sont formés d'un matériau capable de subir une déformation élastique, et la tension sur le support est maintenue après l'augmentation de taille souhaitée.

10. Procédé selon la revendication 8, dans lequel le support et les puits sont formés d'un matériau capable de subir une déformation plastique, et la tension sur le support est relâchée après l'augmentation de taille souhaitée.
